# EUROPÄISCHE PATENTSCHRIFT

(11) **EP 2 443 455 B1**
(45) Veröffentlichungstag und Bekanntmachung des Hinweises auf die Patenterteilung: **02.10.2013**
(21) Anmeldenummer: 10737791.3
(22) Anmeldetag: 21.06.2010
(51) Int. Cl.: G01N 33/53, G01N 33/12

(54) **NEUES VERFAHREN ZUR ISOLATION VON TRICHINELLA ODER ANDEREN PARASITEN AUS ORGANISCHEM GEWEBE**
NOVEL METHOD FOR ISOLATING TRICHINELLA OR OTHER PARASITES FROM ORGANIC TISSUE
NOUVEAU PROCÉDÉ D'ISOLEMENT DE TRICHINELLA OU AUTRES PARASITES DANS DES TISSUS ORGANIQUES

(30) Priorität: 19.06.2009 DE 102009025542; 06.05.2010 CH 700102010
(43) Veröffentlichungstag der Anmeldung: 25.04.2012
(73) Patentinhaber: Bauer, Philipp, 8345 Adetswil (CH)
(72) Erfinder: Bauer, Philipp, 8345 Adetswil (CH)
(74) Vertreter: Vossius & Partner
(86) Internationale Anmeldenummer: PCT/EP2010/058751
(87) Internationale Veröffentlichungsnummer: WO 2010/146184

(56) Entgegenhaltungen:
- US-A- 3 892 529
- US-A- 4 762 789

## Beschreibung

Die vorliegende Erfindung betrifft ein Verfahren zum Nachweis von im Wesentlichen intakten kapselbildenden oder nicht-kapselbildenden Parasiten in Fleisch, das die Mazeration des Fleisches mit einer basischen Verdauungslösung umfasst, die im basischen Milieu aktive Proteasen enthält, wobei diese vorzugsweise eine Endopeptidase, insbesondere eine Serin-Endopeptidase ist und wobei die Verdauungslösung einen pH-Wert zwischen pH 7,3 und 10 hat. Des Weiteren betrifft die vorliegende Erfindung ein Verfahren zum Nachweis von im Wesentlichen intakten kapselbildenden oder nicht-kapselbildenden Parasiten in Fleisch, das die Mazeration des Fleisches mit einer basischen Verdauungslösung umfasst, die z.B. eine Serin-Endopeptidase, wie z.B. Alcalase®, enthält, optional umfassend die Schritte (a) das mechanische Zerkleinern des zu untersuchenden Fleisches; (b) die Mazeration des zu untersuchenden Fleisches durch Zugabe einer Verdauungslösung; (c) Mazeration unter gleichzeitiger Bewegung des Verdauungsansatzes und/oder gleichzeitiger Ultraschall-behandlung; (d) Inaktivierung der Mazeration; (e) Filtration des Mazerats; und (f) Kontrolle, Nachweis, Diagnostik und/oder Typisierung auf Befall mit Parasiten. Des Weiteren sind Verwendungen von im basischen Milieu aktiven Verdauungsenzymen, z.B. einer Serin-Endopeptidase in einem Verfahren zum Nachweis von im Wesentlichen intakten kapselbildenden oder nicht-kapselbildenden Parasiten in Fleisch . Die vorliegende Erfindung stellt auch Serin-Endopeptidasen in der Verwendung in einem Diagnoseverfahren zum Nachweis von im Wesentlichen kapselbildenden oder nicht-kapselbildenden Parasiten in Fleisch bereit. Schließlich wird auch ein Kit offenbart, das die im basischen Milieu aktiven Enzyme und/oder eine basische Verdauungslösung umfasst. Auch die Verwendung der im basischen Milieu aktiven Enzyme und/oder der basischen Verdauungslösungen in Diagnoseverfahren von Infektionen mit intakten kapselbildenden oder nicht-kapselbildenden Parasiten ist beschrieben.

Trichinen (*Trichinella*) sind eine Gattung Fadenwürmer des Stammes *Nematoda* mit parasitischer Lebensweise. Säugetiere, Vögel, Amphibien, Reptilien, Fische und Menschen dienen als Zwischen- und Endwirt. Hauptüberträger auf den Menschen sind Tiere, deren Fleisch durch Trichinen infiziert ist bzw. deren rohes oder ungenügend gekochtes Fleisch, insbesondere von Schweinen, Pferden und Wild. Durch Kochen oder große Kälte können Trichinen abgetötet werden.

Die Trichinen sind durch mehrere Arten weltweit verbreitet. Es gibt acht Arten, wobei die wichtigsten in Europa vorkommenden *Trichinella spiralis, Trichinella britovi, Trichinella pseudospiralis,* und *Trichinella nativa* sind. Andere Arten sind *Trichinella murrelli* (insbesondere in nearktischen Regionen vorkommend), *Trichinella nelsoni* (insbesondere in Äthiopien vorkommend), *Trichinella papuae* (insbesondere in Papua Neuguinea vorkommend) und *Trichinella zimbabwensis* (insbesondere in Zimbabwe vorkommend). *Trichinella spiralis, Trichinella britovi, Trichinella murrelli, Trichinella nelsoni* und *Trichinella nativa* haben Säugetiere als Wirt. *Trichinella pseudospiralis* hat Säugetiere und Vögel als Wirt. *Trichinella papuae* und *Trichinella zimbabwensis* haben Säugetiere und Reptilien als Wirt. Der parasitäre Nematode *Trichinella* spp. ist in der Lage, viele verschiedene Arten zu infizieren, darunter Menschen, Schweine, Ratten, Bären, Pferde und Vögel. In westlichen Ländern Europas treten Trichinen vorwiegend im "silvatischen Zyklus" auf, bei dem Füchse und Nager durch Verspeisen infizierter Tiere die Würmer verbreiten. In nördlichen Gebieten können auch Bären, Schlittenhunde und Robben als Zwischenwirt dienen. Ein "urbaner Zyklus" ist ebenfalls ausgeprägt; bei diesem werden die Erreger überwiegend durch Ratten und Schweine verbreitet.

Die adulten Trichinellen erreichen eine Länge von bis zu 4 mm (Weibchen) bzw. 1,5 mm (Männchen). Deutlich kann man das verdickte Hinterende erkennen, welches den Darm beherbergt. Die Larven encystieren sich im Muskelgewebe und bilden dort einen "Ammenzellnährkomplex", eine Kapsel, die reichlich mit Blutgefäßen versorgt wird und dadurch die Larve am Leben erhält. Sie erreicht eine Größe von rund einem Millimeter und ist hoch infektiös. Es existieren jedoch auch Varianten, die keine Kapsel bilden, wie z. B. *Trichinella pseudospiralis.*

Aufgenommene Muskelzysten werden im Dünndarm aufgelöst und so die Larven freigesetzt. Die Larven bohren sich in das Dünndarmepithel ein und entwickeln sich innerhalb von 30 Stunden zum adulten Tier, danach findet die Paarung statt. Im Dünndarm bringen die Weibchen lebendgebärend bis zu 1500 Larven zur Welt. Die Larven bohren sich anschließend durch den Dünndarm und erreichen so die Lymphe oder den Blutstrom. Sie treiben durch den Körper und lassen sich vor allem im quergestreiften gut durchbluteten Muskelgewebe nieder. Bevorzugt befallenes Gewebe ist das Zwerchfell, die Kaumuskulatur und die Zunge. Dort beginnt die Bildung des "Ammenzellnährkomplexes", der infektiös bleibt, solange der Parasit lebt. Ab dem fünften Monat findet im menschlichen Gewebe eine Verkalkung statt, wobei die encystierten Muskeltrichinen vermutlich noch 5 bis 10 Jahre lebensfähig bleiben. Bei einer Inkubationszeit von 8 bis 15 Tagen entwickeln sich aus den zunächst sich im Dünndarm befindlichen Larven adulte Würmer. Dabei treten neben oft asymptomatischen Krankheitsverläufen auch allgemeine Schwäche, Bauchschmerzen, Übelkeit, Erbrechen und Durchfall auf; nach 1 bis 3 Wochen dann Fieber, Muskelschmerzen und Ödeme im Augenbereich. Diese Symptome halten meist bis zu einem Jahr an und verschwinden danach ohne bleibende Folgen. Als Komplikation kann der Herzmuskel befallen werden, wodurch die Wurminfektion einen tödlichen Verlauf nehmen kann. Das durch Trichinen hervorgerufene Krankheitsbild wird als *Trichinellose* bezeichnet. Die Infektionen unterliegen in der Europäischen Union der Meldepflicht und sind nach dem Infektionsschutzgesetz in Deutschland meldepflichtig.

Wichtigste vorbeugende Maßnahme ist die gesetzlich vorgeschriebene Trichinenschau, die auch als Trichinenuntersuchung bezeichnet wird. Darunter versteht man eine mikroskopische Untersuchung von Fleisch auf Trichinen nach der Schlachtung, wobei die Kapseln der Larven oder die Larven gezielt erkannt werden. Dabei unterliegt für den Verzehr durch Menschen bestimmtes Fleisch von Hausschweinen, Einhufern, Wildschweinen, Bären, Füchsen, Biberratten, Nutrias und Dachsen, sowie allen anderen Tieren, die Träger von Trichinen sein können, der Untersuchungspflicht (untersuchungspflichtige Schlachttiere).

Man unterscheidet im Wesentlichen zwei Methoden, um *Trichinella* spp. in Tieren nachzuweisen. Die erste Methode wird Trichinoskopie oder trichinoskopische Untersuchung genannt. Bei dieser direkten Nachweismethode wird das Gewebe der Trichinenproben, das aus den Zwerchfellpfeilern und aus der Vorderlaufmuskulatur für die Untersuchung entnommen wird, in einem sogenannten Kompressorium (d.h. Quetschglas, bestehend aus zwei Glasplatten) gepresst und anschließend mikroskopisch untersucht. Diese Methode ist wenig sensitiv und arbeitsintensiv. Außerdem können mit dieser Methode keine kapsellosen (nicht-kapselbildenden) Trichinen identifiziert werden. Die zweite Methode ist eine mechanisch unterstützte Methode der künstlichen Verdauung. Dabei wird das die Larven umgebende Muskelgewebe mittels Imitation der natürlichen Verdauung unter Zusatz von Pepsin im sauren Milieu künstlich verdaut, um die Larven dabei freizusetzen. Anschließend werden die Larven mikroskopisch untersucht. Die Trichinenschau wird durch die "Verordnung VO (EG) Nr. 2075/2005 der Kommission vom 5. Dezember 2005 mit spezifischen Vorschriften für die amtlichen Fleischuntersuchungen auf Trichinen" geregelt. Das im Stand der Technik verwendete Verfahren, in dem die natürliche Verdauung unter Zusatz des Enzyms Pepsin, einer Aspartat-Endopeptidase, imitiert und die Verdauung im sauren Milieu durchgeführt wird, verwendet die kommerziell verfügbare Pepsin-Form, die aus dem Magen des Schweins gewonnen wird.

Die im Stand der Technik verwendeten Verfahren sind, wie voran erwähnt, durch die "Verordnung VO (EG) Nr. 2075/2005 der Kommission vom 5. Dezember 2005 mit spezifischen Vorschriften für die amtlichen Fleischuntersuchungen auf Trichinen", geregelt. Die Referenznachweismethode ist das Magnetrührverfahren. Die der Referenznachweismethode gleichwertigen Nachweisverfahren sind: (A) mechanisch unterstützte Methode (Sedimentationstechnik); (B) mechanisch unterstützte Methode ("On-Filter-Isolation"-Technik); (C) automatisches Verdauungsverfahren mit dem Trichomaten; sowie trichinoskopische Untersuchung (Kompressorium). Die Referenznachweismethode und die dieser Referenznachweismethode gleichwertigen Nachweisverfahren sind in der "Verordnung VO (EG) Nr. 2075/2005 der Kommission vom 5. Dezember 2005 mit spezifischen Vorschriften für die amtlichen Fleischuntersuchungen auf Trichinen" beschrieben. Alle im Stand der Technik beschriebenen Nachweisverfahren werden in einer sauren Verdauungslösung durchgeführt. Bei der trichinoskopischen Untersuchung (Kompressorium; "Trichinoskopie") handelt es sich um eine rein mechanische Methode (Pressen zwischen zwei Glasplatten).

Neuere ELISA-basierte Methoden sind sensitiver, sind jedoch bis jetzt nicht für individuelle Schlachtkörperuntersuchungen gemäß "Verordnung VO (EG) Nr. 2075/2005 der Kommission vom 5. Dezember 2005 mit spezifischen Vorschriften für die amtlichen Fleischuntersuchungen auf Trichinen" zugelassen. Außerdem dauert es bei der ELISA-Methode sehr lange, bis die Resultate vorliegen. Zudem sind ELISA-basierende Verfahren auf eine Serokonversion des Wirts angewiesen, bevor ein entsprechender Nachweis erfolgen kann. Diese Serokonversion beansprucht jedoch eine gewisse Zeit (in der Regel 18 Tage bis 5 Wochen).

Um eine Zulassung zur Routineuntersuchung erhalten zu können, muss eine ähnliche Sensitivität und Vergleichbarkeit zu gängigen Untersuchungsmethoden der bereits validierten und zugelassenen Verfahren gegeben sein. Neu zugelassene Methoden müssen unter Wahrung der Rechtsgleichheit eine Alternative zu bereits zugelassenen Methoden darstellen.

Gemäß Bestimmungen der EU soll die Trichinoskopie nicht mehr als Standarduntersuchungsmethode zugelassen sein.

Ein Verdauverfahren zur Freisetzung und Bestimmung von *Trichinella* im Fleisch ist beschrieben, bei dem das Fleisch in einem Mixer im sauren bis neutralen Bereich (pH 2.0 bis 7.2) unter Zugabe von Pepsin oder Bromelin, Trypsin oder Papain zerkleinert und die resultierende Lösung zur anschließenden Analyse sedimentiert wird. Die Unterstützung des Verfahrens durch den Einsatz von Ultraschall, eines Magnetrührers, durch Variation der Temperatur und unter Bewegung ist beschrieben (siehe US 3 892 529 A).

Ferner beschreiben van Knapen *et al.* (siehe van Knapen, F.; Tijdschrift voor diergeneeskunde, 1987, 112: 1095-1100) vergleichende Studien zum Nachweis boviner Sarcosporidiosis. Dabei wird das zu untersuchende Fleisch unter Verwendung von Trypsin im neutralen Bereich artifiziell verdaut. Anschließend wird das Sediment makroskopisch auf Infektion mit *Sarcocystis* spp. untersucht.

Die Isolierung von Nukleinsäuren aus biologischen Proben, die intaktes Gewebe beinhalten, ist im Stand der Technik beschrieben, wobei in den biologischen Proben auch Mikroorganismen, Bakterien und Viren enthalten sein können (siehe US 2005/009 045 A1). Dabei werden die Zellen unter Zugabe eines kationischen Tensids und einer Protease so lange in einem Puffer, der vorzugsweise einen pH von 5.0 bis 7.0 hat, inkubiert, bis die Nukleinsäuren freigesetzt werden. Die freigesetzte Nukleinsäure wird anschließend isoliert. Dabei beschriebene Proteasen sind Subtilisine, Subtilasen und alkalische Serinproteasen. Ferner ist im Stand der Technik ein diagnostisches Reagens beschrieben, das Biopartikel enthält und zur Probenherstellung für Positivkontrollen für Nukleinsäurenachweisverfahren verwendet wird (siehe WO 2009/144 132 A1). Das beschriebene Reagens kann Enzyme wie Proteinase K oder Subtilisine enthalten, wobei im Anschluss an die Lyse der Biopartikel, wie Viren, Bakterien, Protozoen oder Pilze, die freigesetzten Nukleinsäuren isoliert werden.

WO 02/33 129 A2 beschreibt Positivkontrollmaterial zur Amplifikation von Nukleinsäuren, wobei das positive Kontrollmaterial Mikroorganismen in einer biologischen Probe enthält. Dabei ist insbesondere ein Verfahren beschrieben, bei dem nicht-pathogene Mikroorganismen hergestellt werden, indem die Oberflächenproteine der Mikroorganismen partiell mit Enzymen wie Papain, Chymotrypsin, Trypsin oder Pepsin verdaut bzw. modifiziert werden. Der Mikroorganismus, der in eine nicht-pathogene Form überführt wird, ist ein intrazellulärer Parasit.

Ein Verfahren zur Bestimmung von Bakterien in Proben wie Milch oder homogenisiertem Fleisch unter Zugabe von Detergens und proteolytischer Enzyme wie Subtilisine ist in US 5 798 221 A beschrieben, wobei das Ausgangsmaterial des Verfahrens in einem flüssigen Zustand ist. Dabei werden insbesondere somatische, eukaryotische Zellen vollständig lysiert und degradiert und die Proteinpartikel und Zelltrümmer dieser somatischen Zellen aufgelöst, um selektiv die Bakterien in der Probe nachweisen zu können.

Die derzeit verwendeten Untersuchungsmethoden zum Nachweis von *Trichinella* haben eine Reihe von Nachteilen. Alle Verfahren, die auf künstlicher Verdauung im sauren Bereich beruhen, sind relativ zeit- und arbeitsaufwändig. Besonders die Materialkosten, die für die Untersuchung eingesetzt werden müssen, sind recht hoch. Dies ist insbesondere auf den hohen Preis des Enzyms Pepsin zurückzuführen, das in großen Mengen zu der oben erwähnten künstlichen Verdauung im sauren Bereich zugegeben werden muss. Aufgrund der weltweiten Knappheit von Pepsin sind die Preise für dieses Enzym stark angestiegen. Ob langfristig der Bedarf an Pepsin geweckt werden kann, ist unsicher. Versorgungsengpässe könnten dazu führen, dass die gesetzlich vorgeschriebene Trichinenkontrolle nicht mehr durchgeführt werden kann. Ein weiterer Nachteil besteht darin, dass Pepsin zunächst in Pulverform vorliegt und dadurch das technische Personal beim Durchführen der oben beschriebenen gängigen Tests, d.h. beim Versuchsaufbau und bei der eigentlichen Durchführung der oben beschriebenen gängigen Verfahren, dauerhaft der schädlichen und toxischen Wirkung der Pulverstäube exponiert ist. Zudem besteht beim gängigen Verdau im sauren Bereich, vorzugsweise mit Pepsin, der Nachteil, dass die nachzuweisenden Parasiten, also beispielsweise Trichinen, überleben und somit nach wie vor infektiös sein können. Dies impliziert ein potentielles Infektionsrisiko, insbesondere für die oben beschriebenen Personen, die dauerhaft diesen infektiösen Parasiten bei der Durchführung der Nachweisverfahren exponiert sind. Bei den gängigen Verfahren wird obligat Salzsäure (HCl) verwendet. Chlorwasserstoff ist ein farbloses, stechend riechendes Gas, ist ätzend und in hohen Konzentrationen giftig. Beim Einatmen können Reizungen der Schleimhäute und der Atemwege auftreten, die zu einer akuten Bronchitis oder Lungenentzündung führen können. Salzsäure ist ätzend und der Hautkontakt ist unbedingt zu vermeiden. Somit stellt die dauerhafte Exposition mit der obligat einzusetzenden HCl bei der Durchführung der oben beschriebenen Versuche eine weitere Gefahr dar.

Aufgabe der vorliegenden Erfindung ist es, alternative und vorteilhafte Verfahren und Mittel zur Isolation von *Trichinella* oder anderen, insbesondere kapselbildenden, Parasiten aus organischem Gewebe bereitzustellen.

Das technische Problem wird durch die Bereitstellung eines Verfahrens zum Nachweis von im Wesentlichen intakten kapselbildenden oder nicht-kapselbildenden Parasiten in Fleisch gelöst, wobei dieses Verfahren die Mazeration des Fleisches in einer basischen Verdauungslösung umfasst, wobei die Verdauungslösung einen pH-Wert zwischen pH 7,3 und 10 hat. Die basische Verdauungslösung enthält ebenfalls ein im basischen Milieu aktives Verdauungsenzym, wie eine Endopeptidase, zum Beispiel eine Serin-Endopeptidase, wie z.B. Alcalase®. Überraschenderweise wurde gezeigt, dass im basischen Bereich eine Verdauungslösung, die ein solches im basischen Milieu aktives Verdauungsenzym (vorzugsweise Alcalase® (Subtilisin Carlsberg)) enthält, in der Lage ist, ohne erkennbare Nachteile gegenüber dem im Stand der Technik verwendeten Verfahren, die Mazeration des zu untersuchenden Fleisches zu erzielen, um kapselbildende oder nicht-kapselbildende Parasiten im Fleisch nachzuweisen. Der Trichinennachweis per se der vorliegenden Erfindung ist überraschenderweise, wie u.a. in den nachfolgenden Beispielen illustriert, mindestens gleichwertig zu dem im Stand der Technik beschriebenen Verfahren. Dies ist umso überraschender, da im Stand der Technik bisher nur Verfahren beschrieben sind, die Fleisch im sauren Bereich durch Pepsin verdauen. Dabei versucht man, die Vorgänge im Magen zu imitieren, wobei man weiß, dass die Vorgänge im Magen natürlicherweise auf die Verdauung u. a. von stark eiweißhaltigen Nahrungsmitteln wie Fleisch optimiert sind. Im Magen wird dabei insbesondere der Nahrungsbrei mit dem Magensaft vermengt, der im Wesentlichen aus dem eiweißspaltenden Enzym Pepsin und Salzsäure besteht. Durch die Belegzellen des Magens wird Salzsäure produziert. Diese hat nach einer halben bis einer Stunde den gesamten Mageninhalt durchsäuert. Die Säure macht das Enzym Amylase unwirksam, tötet mit der Nahrung eingedrungene Krankheitserreger ab und denaturiert Proteine. Die Hauptzellen sondern das inaktive Enzym Pepsinogen ab, welches durch die Salzsäure zu Pepsin aktiviert wird. Das Pepsin spaltet Proteine in kleinere Peptide, welche später weiter zerlegt werden. Pepsin kann auch Kollagen, den Hauptbestandteil des Bindegewebes, umwandeln. Der pH-Wert im Magen liegt durch die sezernierte Salzsäure bei etwa 0,8.

Wie in den Beispielen illustriert, wird überraschenderweise gezeigt, dass der Nachweis von im Wesentlichen intakten kapselbildenden oder nicht-kapselbildenden Parasiten in Fleisch durch die Mazeration des Fleisches mit einem im basischen Milieu aktiven Verdauungsenzym (hier eine Serin-Endopeptidase) in einer basischen Verdauungslösung möglich ist. Entgegen dem bisherigen Stand der Technik wird also überraschenderweise gezeigt, dass im basischen Bereich eine Serin-Endopeptidase, vorzugsweise Alcalase® (Subtilisin Carlsberg), in der Lage ist, ohne erkennbare Nachteile gegenüber dem im Stand der Technik verwendeten Verfahren, die Mazeration des zu untersuchenden Fleisches zu erzielen, um (intakte) kapselbildende oder nicht-kapselbildende Parasiten im Fleisch nachzuweisen. Ein weiterer Vorteil ist es, dass die verwendeten Enzyme, insbesondere die Serin-Endopeptidase, vorzugsweise Alcalase®, günstiger sind als die im Stand der Technik beschriebenen Enzyme, wie z. B. Pepsin.

Die Lösung des technischen Problems durch die hier offenbarten Verfahren und Verwendungen ist auch insofern überraschend, da US 5 798 221 A lehrt, dass durch Verwendung von Subtilisinen (Enzyme, die im basischen Bereich aktiv sind) somatische, eukaryotische Zellen vollständig lysiert und degradiert und die Proteinpartikel und Zelltrümmer dieser somatischen Zellen aufgelöst werden. Dabei bleiben selektiv die Bakterien in der Probe intakt, die so nachgewiesen werden können. Basierend auf dieser Lehre hätte der Fachmann erwartet, dass auch nachzuweisende Parasiten (die aus somatischen, eukaryotischen Zellen bestehen), im basischen Bereich unter Zugabe von Subtilisin vollständig lysiert und degradiert würden.

Die Lösung des technischen Problems durch die Ausführungsformen dieser Erfindung weist eine Reihe von Vorteilen gegenüber dem im Stand der Technik beschriebenen Verfahren auf. So kann auf die im Stand der Technik verwendete HCl verzichtet werden, da das erfindungsgemäße Verfahren im basischen Bereich durchgeführt wird. Dies hat den Vorteil, dass die dauerhafte Exposition mit der im Stand der Technik eingesetzten HCl und damit potentielle Gefahren für das technische Personal beim Durchführen der oben beschriebenen gängigen Tests, d.h. beim Versuchsaufbau und bei der eigentlichen Durchführung der oben beschriebenen gängigen Verfahren und die damit verbundenen dauerhaften und toxischen Schädigungen vermieden werden können. Ebenso wird auch dauerhaft die schädliche und toxische Wirkung der Pepsin-Pulverstäube bei der Versuchsvorbereitung und Versuchsdurchführung (Pepsin liegt zunächst in Pulverform vor) vermieden, da Subtilisin, im Gegensatz zu Pepsin, in flüssiger Form vorliegt.

Die Lösung des technischen Problems durch die Ausführungsformen dieser Erfindung weist zudem den Vorteil gegenüber dem im Stand der Technik beschriebenen Verfahren auf, dass die nachzuweisenden Parasiten, also beispielsweise Trichinen, nicht überleben und somit nicht mehr infektiös sind. Somit wird ein potentielles Infektionsrisiko vermieden.

Ein weiterer Vorteil des erfindungsgemäßen Verfahrens gegenüber dem im Stand der Technik beschriebenen Verfahren ist es, dass, wie in den Beispielen illustriert, die Verdauung mit Alcalase® bedeutend schneller erfolgt als ein Verdau mit Pepsin.

Somit betrifft die vorliegende Erfindung ein Verfahren zum Nachweis von im Wesentlichen intakten kapselbildenden oder nicht-kapselbildenden Parasiten in Fleisch, das die Mazeration des Fleisches mit einer basischen Verdauungslösung umfasst, die z.B. eine Serin-Endopeptidase enthält, wobei die Verdauungslösung einen pH-Wert zwischen pH 7,3 und 10 hat.

Der Begriff "im Wesentlichen intakt", wie hierin verwendet, bezieht sich vorzugsweise auf die im Verfahren nachzuweisenden kapselbildenden oder nicht-kapselbildenden Parasiten. Diese sind "im Wesentlichen intakt", wenn die Morphologie der nachzuweisenden Parasiten makroskopisch sichtbar bzw. erkennbar ist. Dabei können die Parasiten vollständig intakt sein. Der Begriff bezieht sich aber nicht nur auf vollständig intakte Parasiten, sondern umfasst auch Fragmente der Parasiten, vorzugsweise der Trichinen und/oder Bruchstücke der Kapseln. Dabei sind insbesondere Fragmente umfasst, die für den Fachmann als Bruchstücke bzw. Fragmente der nachzuweisenden Parasiten erkennbar sind. In einer besonderen Ausführungsform umschreibt der Begriff "im Wesentlichen intakt", wenn zwischen ca. 5% und 100% der Morphologie des Gesamtorganismus der nachzuweisenden Parasiten makroskopisch sichtbar bzw. erkennbar sind. In einer weiteren Ausführungsform sind 50%, 60%, 65% 75%, 80%, 85%, 90%, 95%, 97,5%, 98% oder 99% der Morphologie des Gesamtorganismus der nachzuweisenden Parasiten makroskopisch sichtbar bzw. erkennbar.

Der Begriff "Verdauungslösung", wie hierin verwendet, ist dem Fachmann allgemein bekannt und umfasst ein homogenes Gemisch, das aus mindestens zwei chemischen Stoffen besteht und wie hierin verwendet und im Nachfolgenden beschrieben, zur Verdauung des Fleisches dient, um im Wesentlichen intakte kapselbildende oder nicht-kapselbildende Parasiten nachzuweisen. Eine Lösung besteht aus einem oder mehreren gelösten Stoffen (Solute) und einem Lösungsmittel, das in der Regel flüssig ist. Eine "basische Verdauungslösung" ist dem Fachmann allgemein bekannt und umfasst somit eine wässrige Lösung, die in der Lage ist, Hydroxid-Ionen (OH⁻) zu bilden. In einer bevorzugten Ausführungsform umfasst diese basische Verdauungslösung ein oder mehrere eingesetzte Enzyme, wobei der pH-Bereich so gewählt ist, dass er im pH-Optimum der jeweils eingesetzten Enzyme liegt.

Dem Fachmann ist allgemein bekannt, dass der pH ein Maß für die saure oder alkalische Reaktion einer wässrigen Lösung ist. Der pH-Wert ist eine dimensionslose Zahl, die den pH charakterisiert. Der pH-Wert ist definiert als der negative dekadische Logarithmus der Wasserstoffionen-Aktivität. In Anlehnung an die Dissoziationskonstante des Wassers, k_{Diss} = c(H⁺) · c(OH⁻) = 10⁻¹⁴ Mol²/Liter², teilt man die Wertebereiche für reines Wasser und verdünnte wässrige Lösungen bei 22 °C ein in: pH < 7 entspricht einer Lösung mit saurer Wirkung; pH = 7 entspricht absolut reinem Wasser oder einer neutralen Lösung; pH > 7 entspricht einer alkalischen Lösung (basische Wirkung). In den meisten wässrigen Lösungen liegen die pH-Werte zwischen 0 (stark sauer) und 14 (stark alkalisch).

Dem Fachmann sind eine Vielzahl an Methoden bekannt und vertraut, um den pH-Wert einer Lösung mit unterschiedlichen Methoden zu ermitteln, z. B. elektronische Verfahren, mittels Potentiometrie oder mittels Indikatorfarbstoffen, wie z. B. Lackmus, Phenolphthalein, Methylorange oder Bromthymolblau. Dem Fachmann sind weitere Verfahren zur pH-Wert-Bestimmung geläufig und bekannt.

Gemäß der Erfindung umfasst die "basische Verdauungslösung" eine Verdauungslösung, in der das eingesetzte (Verdauungs-)Enzym aktiv ist und seine Enzymaktivität im basischen Bereich entfaltet. Das heißt, dass je nach eingesetztem Enzym, ein basischer pH-Bereich gewählt ist, in dem das pH-Optimum des jeweils eingesetzten Enzyms liegt. Dabei ist dem Fachmann allgemein bekannt, dass der pH-Wert einen entscheidenden Einfluss auf die Enzymkinetik und somit auf den zeitlichen Verlauf enzymatischer Reaktionen hat. Eine zentrale Größe hierbei ist die Reaktionsgeschwindigkeit. Sie ist ein Maß für die Änderung der Substratkonzentration mit der Zeit, also für die Stoffmenge Substrat, die in einem bestimmten Reaktionsvolumen pro Zeiteinheit umgesetzt wird (Einheit: mol/(l·s)). Neben den Reaktionsbedingungen wie Temperatur und Salzkonzentration der Lösung, Konzentrationen des Enzyms und der Substrate und Anwesenheit von Effektoren (Aktivatoren oder Inhibitoren), hängt die Reaktionsgeschwindigkeit insbesondere vom pH-Wert der Lösung ab. Wie voran erwähnt, steht im Zusammenhang mit der Reaktionsgeschwindigkeit die Enzymaktivität. Sie gibt an, wie viel aktives Enzym sich in einer Enzym-Präparation befindet. Die Einheiten der Enzymaktivität sind Unit (U) und Katal (kat), wobei 1 U definiert ist als diejenige Menge Enzym, welche unter angegebenen Bedingungen ein Mikromol Substrat pro Minute umsetzt: 1 U = 1 µmol/min. Änderungen im pH-Wert der Lösung haben oft dramatische Effekte auf die Enzymaktivität, da der pH-Wert die Ladung einzelner für die Katalyse wichtiger Aminosäuren im Enzym beeinflussen kann. Jenseits des pH-Optimums vermindert sich die Enzymaktivität und kommt irgendwann zum Erliegen. Ähnliches gilt für die Salzkonzentration bzw. die Ionenstärke in der Umgebung.

Der Begriff "Fleisch" umfasst dabei z.B. Fleisch nach der Schlachtung von Schlachttieren, aber auch Fleisch von zu präparierenden Tieren, oder auch Fleischproben zur in vitro Diagnostik von Infektionen mit intakten kapselbildenden oder nicht-kapselbildenden Parasiten. Wie hierin verwendet, bezieht sich der Begriff "Fleisch" insbesondere auf Fleisch von untersuchungspflichtigen Schlachttieren, wobei der Begriff "untersuchungspflichtige Schlachttiere" dem Fachmann allgemein bekannt ist. Im weiteren Sinne kann das zu untersuchende Fleisch aber auch von Säugern, Fischen, Reptilien, Vögeln oder Amphibien stammen. Somit kann das zu untersuchende Fleisch z.B. von Hausschweinen, Rehen, Rindern, Hirschen, Gämsen, Elchen, Wildschweinen, Einhufern, Bären, Füchsen, Biberratten (Nutria), Marderhunden (*Nyctereutes procyonoides*), Straussenvögeln, Krokodilen, Pferden sowie Schlachtvieh bzw. Schlachttieren, Haustieren und Wildtieren wie Haarwild, Federwild, Schalenwild, Schwarzwild, Hochwild, Niederwild, Rehwild, Raubwild, Großwild, Ballenwild oder auch Dachsen stammen. Außerdem kann das zu untersuchende Fleisch auch in Übereinstimmung mit der vorliegenden Erfindung das Fleisch von Fischen umfassen. Beispielsweise ist bekannt, dass auch Heringe von Parasiten befallen sein können. Das zu untersuchende Fleisch von Fischen kann aber nicht nur von Heringen stammen, sondern von allen, dem Fachmann bekannten Fischen. Das Fleisch kann aber auch von allen anderen Tieren stammen, die von kapselbildenden oder nicht-kapselbildenden Parasiten infiziert sein können. Vorzugswiese kann das Fleisch also auch von anderen Tieren stammen, die Träger von Parasiten des Stammes der Nematoda (Fadenwürmer) sind, insbesondere Träger von Trichinen, wie Trichinella, bzw. von diesen infiziert sind. Außerdem kann das zu untersuchende Fleisch auch in Übereinstimmung mit der vorliegenden Erfindung das Fleisch umfassen, das durch Jagd von Wild erhalten wird. "Wild" in Übereinstimmung mit der vorliegenden Erfindung umfasst die Gesamtheit der auf der Erde vorkommenden jagdbaren Tierarten. Insbesondere umfasst der Begriff "Fleisch" Fleisch von Tierpopulationen, die für den Menschen zum Verzehr geeignet sind. Das zu untersuchende Fleisch stammt vorzugsweise von schon toten, insbesondere geschlachteten, Tieren. Jedoch ist auch hierin die Verwendung eines im basischen Milieu aktiven Verdauungsenzyms, einer Serin-Endopeptidase, oder einem Enzym der Enzymgruppe der Subtilisine, wie Alcalase® (Subtilisin Carlsberg) oder Alcalase 2,5L® zur Verwendung in einem Diagnoseverfahren zum Nachweis einer Infektion mit kapselbildenden oder nicht-kapselbildenden Parasiten im Sinne der vorliegenden Erfindung gemeint. Hier kann das Fleisch auch von noch lebenden Tieren stammen. Das Fleisch kann sowohl Muskelfleisch als auch Fleisch aus anderen Geweben sein. Somit kann das Fleisch aus anderen, nicht rein muskulösen Gewebe, wie z.B. Diaphragma oder Zungenfleisch stammen.

Die Erfindung betrifft ebenfalls ein Verfahren zum Nachweis von intakten kapselbildenden oder nicht-kapselbildenden Parasiten in Fleisch, das die Mazeration des Fleisches mit einer basischen Verdauungslösung umfasst, die ein im basischen Milieu aktives Verdauunesenzym, wie eine Serin-Endopeptidase, wie z.B. Alcalase®, enthält. Dieses Verfahren kann folgende Schritte umfassen: (a) das mechanische Zerkleinern des zu untersuchenden Fleisches; (b) die Mazeration des zu untersuchenden Fleisches durch Zugabe einer Verdauungslösung; (c) Mazeration unter gleichzeitiger Bewegung des Verdauungsansatzes und/oder gleichzeitiger Ultraschallbehandlung; (d) Inaktivierung der Mazeration; (e) Filtration des Mazerats; und (f) Kontrolle, Nachweis, Diagnostik und/oder Typisierung auf Befall mit Parasiten.

Erfindungsgemäß kann das mechanische Zerkleinern des Fleisches auch in Anwesenheit der hierin beschriebenen Verdauungslösung (und/oder des im basischen Bereichs aktiven Verdauungsenzyms) stattfinden.

Der Begriff "Mazeration", wie hierin beschrieben, umschreibt das Verfahren zur Auflösung von organischem Gewebe. Der Begriff umschreibt im klassischen Sinne primär keinen chemischen, sondern einen rein physikalischen Vorgang, da keine chemische Reaktion per se abläuft. "Mazeration" bezieht sich im klassischen Sinne auf ein physikalisches Verfahren, bei dem ein Körper oder Gegenstand einige Zeit der Einwirkung einer Flüssigkeit wie zum Beispiel Wasser, Öl oder Alkohol ausgesetzt wird, welche als Lösungsmittel für bestimmte Inhaltsstoffe dieses Gegenstandes dienen, das Produkt wird als Mazerat bezeichnet. Dabei wird der Gegenstand oder Körper als solcher nicht aufgelöst, sondern nur bestimmte Bestandteile davon gehen in die Flüssigkeit über, die als Lösemittel dient. Vorzugsweise ist der Körper oder Gegenstand als das voran beschriebene Fleisch zu verstehen. Der Begriff "Mazeration", wie hier in der vorliegenden Erfindung beschrieben und verwendet, umfasst aber nicht nur den rein physikalischen Vorgang, sondern beschreibt vielmehr einen ablaufenden (bio-)chemischen Vorgang, bei dem das Fleisch, wie in der nachfolgend beschriebenen Verdauungsreaktion, aufgelöst und/oder zumindest angedaut oder anverdaut wird und dabei bevorzugt die gegebenenfalls vorhandenen Parasiten, wie z.B. Trichinen freisetzt. Im Kontext der vorliegenden Erfindung findet diese physikalische Zerkleinerung des Fleisches, die Mazeration, daher in der hierin beschriebenen basischen Verdauungslösung unter Verwendung eines im basischen Milieu aktiven Verdauungsenzyms, wie einer Serin-Endopeptidase, z.B. Subtilisin/Alcalase®, statt.

Die Mazeration im Sinne dieser Erfindung kann auch ein rein mechanischer Schritt der Zerkleinerung von Fleisch oder Fleischstücken vorangehen.

Wie in den Beispielen illustriert, stellt die vorliegende Erfindung ein Verfahren zum Nachweis von im Wesentlichen intakten kapselbildenden oder nicht-kapselbildenden Parasiten in Fleisch bereit, das die Mazeration des Fleisches mit einer basischen Verdauungslösung umfasst, wobei die Verdauungslösung einen pH-Wert zwischen pH 7,3 und 10 hat, wobei die Verdauungslösung ein im basischen Milieu aktives Verdauungsenzym, insbesondere eine Serin-Endopeptidase, enthält, wobei diese Verfahren folgende Schritte umfassen kann: (a) das mechanische Zerkleinern des zu untersuchenden Fleisches; (b) die Mazeration des zu untersuchenden Fleisches durch Zugabe der basischen Verdauungslösung; (c) Inaktivierung der Verdauungsmazeration; (d) Filtration des Mazerats; und (e) Kontrolle, Nachweis, Diagnostik und oder Typisierung auf Befall mit Parasiten, wobei das Verhältnis Fleisch/Verdauungslösung vorzugsweise 1:10 ist. In einer weiteren Ausführungsform kann das Verhältnis davon abweichen und beispielsweise 1:5 oder 1:20 betragen.

Im erfindungsgemäßen Verfahren wird vorzugsweise eine Serin-Endopeptidase verwendet, wobei das Enzym zur Enzymgruppe der Subtilisine gehört.

Wie voran beschrieben, beruhen die erfindungsgemäßen Verfahren auf der Verwendung von basischen Verdauungslösungen, die, im Basischen aktive Verdauungsenzyme, wie Serinendopeptidasen, umfassen. Diese Enzyme können auch die in den Beispielen verwendeten Enzyme der Enzymgruppe der Subtilisine sein. Der Fachmann kennt jedoch weitere im alkalischen/basischen Milieu aktive Verdauungsenzyme wie alkalische Proteasen, wobei es sich in einer Ausführungsform bei diesen alkalischen Proteasen um Alkalische Aminopeptidase (EC 3.4.11), Alkalische Cystein Peptidase (EC 3.4.22) oder Alkalische Metallopeptidase (EC 3.4.24) handelt. Auch diese können erfindungsgemäß eingesetzt werden, wobei sich die bereitgestellten Beispiele auf die bevorzugt eingesetzten Serinproteasen beziehen. Die Erfindung umfasst jedoch auch die Verwendung von basischen Verdauungslösungen in der Fleischverarbeitung, in welchen mehrere, d.h. mindestens 2 Verdauungsenzyme eingesetzt werden, die im basischen Milieu aktiv sind.

Bei den erfindungsgemäß einzusetzenden, im Basischen aktiven Verdauungsenzymen, können Enzyme, wie Subtilisine, verwendet werden, die rekombinant oder nichtrekombinant hergestellt werden.

Die erfindungsgemäß einzusetzenden Enzyme können Subtilisine sein. Subtilisin kann insbesondere ausgewählt sein aus Alcalase® (Subtilisin Carlsberg) und Alcalase 2,5L®. Alcalase® kann z. B. von der Firma Novozymes A/S, 2880 Bagsvaerd, Dänemark unter dem Produktnamen "Alcalase ® 2,5 L DX (Temperatur 40-50; pH 7.0-9.5)" bezogen werden.

Die in den hier bereitgestellten Verfahren, Verwendungen und Kits zu nutzenden basischen Verdauungsenzyme können vorteilhaft in einer Konzentration von 5 bis 80g pro Liter Mazerationsflotte eingesetzt werden, z.B. 5.0, 10, 15, 20, 25, 30, 35, 40, 45, 50, 55, 60, 65, 70, 75 oder 80g pro Liter. Alcalase® (Subtilisin Carlsberg) wurde insbesondere in den beigefügten Beispielen in einer Konzentration von 20g bis 40g pro Liter Mazerationsflotte eingesetzt, also z.B. 20, 25, 30, 35 oder 40g pro Liter. Der Fachmann kann ohne Weiteres sinnvolle Konzentrationen der in den bereitgestellten Verfahren, Verwendungen und Kits zu nutzenden basischen Verdauungsenzyme ableiten und durch Routineversuche bestimmen.

Erfindungsgemäß wird eine basische Verdauungslösung mit einem pH-Wert zwischen pH 7,3 und 10 verwendet.

Der pH-Wert der Verdauungslösung ist vorzugsweise größer als pH 7.5, 7.8, 8.0 oder 8.5. Wie in den Beispielen gezeigt kann die Verdauungslösung einen pH zwischen pH 7,3 und 9 haben, z. B. pH 8.0 oder pH 8.5. Andere pH-Werte der Verdauungslösung sind z. B. 7.5, 7.8, 8.0, 8.5, 9.0 oder 9.5. In einer weiteren bevorzugten Ausfuhrungsförm wird, je nach eingesetztem Enzym, ein basischer pH-Bereich gewählt, in dem das pH-Optimum des jeweils eingesetzten, im basischen Milieu aktiven Enzyms, liegt.

In einer weiteren Ausführungsform umfasst das vorstehend beschriebene Verfahren eine Verdauungslösung, wobei die Verdauungslösung Salze, wie z. B. Kochsalz (NaCl) enthält.

Insbesondere kann die im erfindungsgemäßen Verfahren eingesetzte Verdauungslösung optional weitere Bestandteile beinhalten, wobei dies Fettemulgatoren, Tenside und/oder andere Hilfsstoffe sein können. In einer weiteren Ausführungsform kann die Verdauungslösung Farbstoffe oder färbende Mittel enthalten, mit denen die nachzuweisenden Parasiten angefärbt werden können. Die auf diese Weise angefärbten Parasiten können den optisch-visuellen Nachweis der Parasiten in den Proben und/oder die Typisierung der Parasiten erleichtern. In einer Ausführungsform umfassen die Fettemulgatoren auch Detergentien. Einige häufig verwendete Detergentien sind Triton X-100^{®}, Triton-X-114^{®}, NP-40^{®}; CHAPS, Tween-20^{®}, Tween-40^{®}, Tween-80^{®}, Octyl Glucoside, Octylthio Glucoside, Brij-35, Brij-58, SDS und ähnliche, wobei diese Liste nicht erschöpfend ist. Man unterscheidet generell zwischen ionischen und nicht-ionischen Detergentien, wobei im Folgenden nur einzelne Beispiele zu den jeweiligen Klassen genannt sind:
Ionische Detergentien:
   Anionische Detergentien (basierend auf Sulfat, Sulfonat oder Carboxylat)
      Perfluorooctanoate (PFOA oder PFO)
      Perfluorooctanesulfonate (PFOS)
      Sodium Dodecyl Sulfate (SDS), Ammonium Lauryl Sulfate, und andere
      Alkyl Sulfat-Salze
      Sodium Laureth Sulfate, auch bekannt als Sodium Lauryl Ether Sulfate
      (SLES)
      Alkyl Benzene Sulfonate
      Seifen oder Fettsäuresalze
   Kationische Detergentien (basierend auf quaternären Ammoniumkationen)
      Cetyl Trimethylammonium Bromide (CTAB) a.k.a. Hexadecyl Trimethyl
      Ammonium Bromide und andere Alkyltrimethylammonium Salze
      Cetylpyridinium chloride (CPC)
      Polyethoxylated tallow amine (POEA)
      Benzalkonium Chloride (BAC)
      Benzethonium Chloride (BZT)
   Zwitterionische Detergentien (amphoteric)
      CHAPS (3-[(3-Cholamidopropyl)dimethylammonio]-1-propanesulfonate)
      Dodecyl betaine
      Cocamidopropyl betaine
      Coco ampho alycinate
Nicht-ionische Detergentien:
   Alkyl poly(ethylene oxide)
      Polysorbates: basierend auf Polyoxyethylene Glycol, einschließlich der Tween Serie (ex. Tween 20, Tween 80), die Brij Serie], die [[ex.Triton detergent[Triton]] Serie (ex.Triton X-100)
   Alkylphenol poly(ethylene oxide)
   Copolymere von Poly(ethylene oxide) und Poly(propylene oxide) (kommerziell bekannt unter dem Namen Poloxamers oder Poloxamines)
   Alkyl polyglucosides, einschließlich:
      Octyl glucoside
      Decyl maltoside
   Fettalkohole:
      Cetylalkohol
      Oleylalkohol
   Cocamide MEA, Cocamide DEA
   Dodecyl Dimethylamine Oxide

Dem Fachmann sind darüber hinaus noch weitere Emulgatoren/Fettemulgatoren und Detergentien bekannt. Ein Detergens ist ein wasserlöslicher, organischer Stoff, der die Wasseroberflächenspannung herabsetzt und Fett bindet. Man versteht darunter sowohl natürlich vorkommende, als auch synthetisch hergestellte Tenside (Emulgatoren und Netzmittel). Der Ausdruck "Detergens" bezeichnet also einen Stoff oder eine Zubereitung, welcher/welche Seifen und/oder andere Tenside enthält und für Wasch- und Reinigungsprozesse bestimmt ist. Detergentien können unterschiedliche Formen haben (Flüssigkeit, Pulver, Paste, Riegel, Tafel, geformte Stücke, Figuren usw.) und Bestandteil der erfindungsgemäßen Verdauungslösung sein. Jedoch können diese Emulgatoren/Fettemulgatoren/Detergentien auch nach Verdau im Basischen zugesetzt werden. Somit kann der Emulgator/Fettemulgator/Detergens sowohl vor, während als auch nach dem Verdau mit einem im basischen Milieu aktiven Enzym zugesetzt und erfindungsgemäß verwendet werden.

In einer erfindungsgemäßen Ausführungsform umfasst die basische Verdauungslösung einen Fettemulgator, wobei der Emulgator/Fettemulgator vorzugsweise Supralan UF® ist. Detergentien, Emulgatoren/Fettemulgatoren usw. können in gängigen Konzentrationen eingesetzt werden. Zum Beispiel kann Supralan UF® in einer Konzentration von ca. 0.1 bis ca. 30g, von ca. 2 bis ca. 20g, von ca. 3 bis ca. 15g, oder von ca. 4 bis ca. 10 g pro Liter Mazerationsflotte eingesetzt werden, z.B. 0.1, 0.2, 0.3, 0.4, 0.5, 0.6, 0.7, 0.8, 0.9, 1.0, 2, 3, 4, 25, 5, 6, 7, 8, 9, 10, 11, 12, 13, 14. 15, 16, 17, 18, 19, 20, 22, 25, 27 oder 30g pro Liter. Supralan UF® wurde insbesondere in den beigefügten Beispielen in einer Konzentration von z.B. 8g pro Liter Mazerationsflotte eingesetzt. Der Fachmann kann ohne Weiteres sinnvolle Konzentrationen der in den bereitgestellten Verfahren, Verwendungen und Kits zu nutzenden basischen Verdauungsenzyme ableiten und durch Routineversuche bestimmen, um , z.B. Ausflockungen wie von Fett, zu verhindern.

Wie voran beschrieben, beruhen die erfindungsgemäßen Verfahren auf dem Einsatz von basischen Verdauungslösungen, die, wie in den Beispielen gezeigt, im Basischen aktive Verdauungsenzyme, wie z.B. Serinendopeptidasen beinhalten. Diese Verdauungslösungen können in einer weiteren Ausführungsform auch einen Emulgator/Fettemulgator enthalten. Somit können erfindungsgemäß Verdauungslösungen eingesetzt werden, um unter Umständen während des erfindungsgemäß durchgeführten Verfahrens auftretende Ausflockungen zu verhindern und/oder abzuschwächen. Dies hat den Vorteil, dass das erfindungsgemäße Verfahren nicht wesentlich behindert wird, sollten diese Ausfällungen auftreten. Somit umfasst die vorliegende Erfindung auch im Hinblick auf die hier bereitgestellten Beispiele eine wie hierin beschriebene basische Verdauungslösung, die ebenfalls einen Emulgator/Fettemulgator beinhalten kann. Eine solche Verdauungslösung umfasst neben den im basischen Milieu aktiven Verdauungsenzymen auch Emulgatoren/Fettemulgatoren.

In einer anderen bevorzugten Ausführungsform enthält die hierin beschriebene basische Verdauungslösung weitere Hilfsstoffe, wobei die Hilfsstoffe ausgewählt sein können aus der Gruppe bestehend aus Coenzymen, Enzymsubstraten, Katalysatoren etc.. Diese Coenzyme, Enzymsubstrate, Katalysatoren etc. sind vorzugsweise ebenfalls im basischen Bereich aktiv.

In Übereinstimmung mit dem Vorstehenden umfasst die vorliegende Erfindung ein Verfahren zum Nachweis von im Wesentlichen intakten kapselbildenden oder nicht-kapselbildenden Parasiten in Fleisch, das die Mazeration des Fleisches mit einer basischen Verdauungslösung umfasst, die eine Serin-Endopeptidase enthält, wobei die Mazeration auch unter gleichzeitiger Ultraschallbehandlung erfolgen kann, wobei die Verdauungslösung einen pH-Wert zwischen pH 7,3 und 10 hat.

In Übereinstimmung mit dem Vorstehenden umfasst die vorliegende Erfindung ein Verfahren zum Nachweis von im Wesentlichen intakten kapselbildenden oder nicht-kapselbildenden Parasiten in Fleisch, das die Mazeration des Fleisches mit einer basischen Verdauungslösung umfasst wobei die Verdauungslösung einen pH-Wert zwischen pH 7,3 und 10 hat, die z.B. eine Serin-Endopeptidase enthält, wobei die Mazeration zum Beispiel unter gleichzeitiger Bewegung und/oder gleichzeitigem Rühren erfolgt. In einer weiteren Ausführungsform erfolgt die Mazeration unter gleichzeitiger Bewegung und/oder gleichzeitigem Rühren mit einem Magnetrührer.

In einer anderen Ausführungsform umfasst das hierin beschriebene Verfahren eine Fleischmazeration/Fleischverdauungsmazeration im basischen Milieu, wobei dieses Verfahren vorzugsweise bei einer Temperatur zwischen 40 und 65 °C durchgeführt wird. In einer weiteren Ausführungsform wird die Mazeration bei einer Temperatur zwischen 55 und 65 °C durchgeführt. In einer weiteren Ausführungsform wird die Mazeration bei einer Temperatur von 55 °C durchgeführt. Die Erfindung ist nicht nur auf die erwähnten Temperaturbereiche beschränkt. Je nach eingesetztem Enzym kann auch ein Temperatur-Bereich gewählt werden, der dem Temperatur-Optimum des jeweils eingesetzten Enzyms entspricht.

Das erfindungsgemäße Verfahren kann auch einen Schritt umfassen, in dem die hierin beschriebene Mazeration durch Filtration der Reaktionslösung und/oder durch Abkühlen der Reaktionslösung terminiert wird. Diese Terminierung der Reaktion, wie hierin beschrieben, ist als Inaktivierung der Reaktion zu verstehen.

In einer Ausführungsform wird die (Verdauungs-)Mazeration durch Filtration terminiert, wobei bei der Filtration des Mazerats die Maschengröße der verwendeten Filter auf die Größe der nachzuweisenden Organismen angepasst werden kann. In einer weiteren Ausführungsform kann die Filtration zur Terminierung der Reaktion gegebenenfalls unter Zusatz von Druck oder Vakuum erfolgen.

Wie in den Beispielen illustriert, kann eine Filtration oder Zwangsfiltration (Filtration der Lösung, gegebenenfalls unter Zusatz von Druck oder Vakuum), durchgeführt werden. Ein entscheidender Vorteil ist dabei ein Zeitgewinn gegenüber einem Verfahren ohne Filtration. Während der Sedimentationsphase kann unter Umständen eine Sedimentation nach unten und ein Aufschwimmen von Material nach oben beobachtet werden. Ausserdem kann die Verdauungsflüssigkeit während der Sedimentation flockig ausfallen. Die Ausfällung kann dann nach oben aufschwimmen und verhindert so eine vollständige Sedimentation der nachzuweisenden Parasiten. Sollte dies vorkommen, dann hat eine Zwangsfiltration einen weiteren positiven Effekt, der darin liegt, dass man dadurch diesem Vorgang des Aufschwimmens entgegenwirken kann. Somit erfolgt eine vollständige Sedimentation der nachzuweisenden Parasiten.

In Übereinstimmung mit dem Vorstehenden umfasst die vorliegende Erfindung ein Verfahren zum Nachweis von kapselbildenden oder nicht-kapselbildenden Parasiten in Fleisch, das die (Verdauungs-)Mazeration des Fleisches mit einer basischen Verdauungslösung umfasst, wobei die Verdauungslösung einen pH-Wert zwischen pH 7,3 und 10 hat, die ein im basischen Milieu aktives (Verdauungs-) Enzym, wie eine Serin-Endopeptidase, umfasst, wobei die anschließende Kontrolle, der anschließende Nachweis, die anschließende Diagnostik und/oder die anschließende Typisierung auf Befall mit Parasiten visuell erfolgt. Allerdings kann der Nachweis von ggf. vorhandenen Parasiten auch durch eine anschließende Kontrolle, einen anschließenden Nachweis, eine anschließende Diagnostik und/oder eine anschließende Typisierung der einschlägigen, parasitären Nukleinsäuremoleküle oder Proteine erfolgen. Der Begriff "anschließend", wie hier verwendet bezieht sich darauf, dass das erfindungsgemäße Verfahren zunächst den Nachwies und/oder die Isolierung von im Wesentlichen intakten kapselbildenden oder nicht-kapselbildenden Parasiten in Fleisch umfasst. Erst diese "im Wesentlichen intakten kapselbildenden oder nicht-kapselbildenden Parasiten" werden, vorzugsweise, anschließend mit molekularbiologischen oder biochemischen Verfahren wie hier beschrieben, untersucht/bestimmt.

In einer Ausführungsform kann der Nachweis der Nukleinsäuremoleküle über eine PCR-Reaktion erfolgen. Dabei ist besonders hervorzuheben, dass die Nukleinsäuremoleküle bei der voran beschriebenen Mazeration möglichst nicht bzw. wenig degradiert werden und möglichst intakt bleiben sollten. In einer Ausführungsform beruht der Nachweis der Nukleinsäuremoleküle auf dem Nachweis entsprechender spezifischer Sequenzen, die diese kapselbildenden oder nicht-kapselbildenden Parasiten eindeutig nachweisen können. Der Fachmann kann geeignete Sequenzen zum sequenzspezifischen Nachweis aus bekannten Datenbanken ableiten. In einer weiteren Ausführungsform soll der Nachweis, die Diagnostik und/oder die Typisierung auf Befall mit Parasiten auf serumbasierten Methoden bzw. serologischen Verfahren beruhen. Diese serologischen/immunologischen Verfahren zum Nachweis von kapselbildenden oder nicht-kapselbildenden Parasiten beruhen in einer Ausführungsform auf dem Nachweis entsprechender Antigene dieser kapselbildenden oder nicht-kapselbildenden Parasiten. Im Falle, dass Antikörper verwendet werden, bildet sich ein Immunkomplex, der durch ein dem Fachmann bekanntes Verfahren nachgewiesen werden kann. In einer weiteren Ausführungsform können weitere serologische/immunologische Verfahren verwendet werden, z. B. Immunoblot-Verfahren, ELISA, RIA, SPR (surface plasmon resonance) oder Agglutinationstests, wobei hier nur einige Beispiele aufgelistet sind.

Wie voran beschrieben, kann in einer besonderen Ausführungsform der Nachweis der Nukleinsäuremoleküle über eine PCR-Reaktion erfolgen. Dabei erfolgt die Amplifikation von Nukleinsäuren durch eine nachfolgend beschriebene, dem Fachmann bekannte PCR-Reaktion (Polymerase Chain Reaction). Andere Amplifizierungsverfahren sind z. B. Ligase Chain Reaction (LCR), gap-filling LCR (Gap-LCR), Nukleinsäuresequenz-basierte Amplifizierung (NASBA) und Transkriptions-vermittelte Amplifizierung (TMA), Diese Verfahren sind im Stand der Technik gut bekannt.

Die PCR-Technik ist z B. umfassend in PCR Technology, Principles and Applications for DNA Amplification, Erlich, Hrsg.(1992); PCR Protocols, A Guide to Methods and Applications, Innis et al., Hrsg (1990); R. K Saiki et al., Science 230: 1 350 (1985) und US 4,683,202 beschrieben, deren Offenbarung vollinhaltlich hier aufgenommen wird. Die Real-Time PCR ist z. B. in EP-A-O 51 2 34, EP-A-O 640 828, EP-A-O 51 9 338 (F. Hoffmann-La Roche AG) beschrieben. Es werden hierfür kommerzielle Systeme angeboten, z. B. TaqMan® (Roche Molecular Systems, Inc., Branchburg Township, New Jersey).

Dem Fachmann sind weitere Standardverfahren zum Nachweis von Nukleinsäuren bekannt oder er kann diese Verfahren von Standardlehrbüchem (z. B. Sambrook, et al., 2001, loc. cit) ableiten.

Wie voran beschrieben, sollen in einer weiteren Ausführungsform der Nachweis, die Diagnostik und/oder die Typisierung der Parasiten auf serumbasierten Methoden bzw. serologischen Verfahren beruhen. Diese serologischen/immunologischen Verfahren zum Nachweis von kapselbildenden oder nicht-kapselbildenden Parasiten beruhen in einer bevorzugten Ausführungsform auf dem Nachweis entsprechender Antigene dieser kapselbildenden oder nicht-kapselbildenden Parasiten. Im Falle, dass Antikörper verwendet werden, bildet sich ein Immunkomplex, der durch ein dem Fachmann bekanntes Verfahren nachgewiesen werden kann. Dabei nutzt man u. a. Immunagglutination. Immunopräzipitation (Immunodiffusion, Immunelektrophorese oder Immunfixierung), Western Blot Techniken (z. B. in situ) Immunhistochemie, (in situ) Immuncytochemie, Affinitätschromatographie, Enzym-Immunoassays). Polypeptide können auch z. B. in Lösung durch physikalische Verfahren, wie z. B. Photometrie bestimmt werden. Verfahren zur Quantifizierung eines bestimmten Polypeptids in einer Mischung sind aufgrund der spezifischen Bindung, z. B. von Antikörpern, möglich. Spezifische Nachweismethoden und Quantifizierungsverfahren nutzen die Spezifität der Antikörper, z. B. in immunhistochemischen Verfahren. So kann z. B. die Konzentration, die Menge oder das Vorkommen bzw. das Fehlen entsprechender spezifischer Antigene der kapselbildenden oder nicht-kapselbildenden Parasiten durch einen enzyme-linked immunosorbant assay (ELISA) bestimmt werden. Alternativ können auch Western Blot Verfahren sowie/oder immunhistochemische Färbeverfahren durchgeführt werden. Western Blot kombiniert z. B. die Auftrennung einer Mischung von Proteinen durch Elektrophorese und den anschließenden spezifischen Nachweis mit Antikörpern. Die Elektrophorese kann auch multi-dimensional sein, wie die 2D-Elektrophorese. Dabei werden Polypeptide gewöhnlich in der 2D-Elektrophorese in einer Dimension nach ihrem Molekulargewicht und in der anderen Richtung nach ihrem isoelektrischen Punkt aufgetrennt. In einer weiteren Ausführungsform können weitere serologische/immunologische Verfahren verwendet werden, z. B. Immunoblot-Verfahren, ELISA, RIA, SPR (surface plasmon resonance) oder Agglutinationstests, wobei hier nur einige Beispiele aufgelistet und dem Fachmann bekannt sind. Diese und andere geeignete Verfahren zum Nachweis und/oder zur Bestimmung der Menge/Konzentration eines spezifischen Proteins/Polypeptides sind dem Fachmann bekannt und z. B. in Sambrook, et al., 2001,, loc. cit. beschrieben.

Der Nachweis, die Diagnostik und/oder die Typisierung auf Befall mit Parasiten kann auf serumbasierten Methoden bzw. serologischen Verfahren wie dem enzyme-linked immunosorbant assay (ELISA) beruhen. Der Begriff "ELISA" bezeichnet ein immunologisches Nachweisverfahren (*Assay*)*,* das im Gegensatz zum Radioimmunassay (RIA) nicht auf einer Radioaktivitätsmessung, sondern auf einer enzymatischen Farbreaktion basiert. Wie der Radioimmunassay gehört auch der ELISA zur Gruppe der Immunassay-Verfahren. Beide Verfahren sind dem Fachmann bekannt und im Stand der Technik beschrieben.

Das erfindungsgemäße Verfahren kann insbesondere zum Nachweis von Parasiten des Stammes der *Nematoda* (Fadenwürmer) eingesetzt werden. In einer Ausführungsform der vorliegenden Erfindung handelt es sich bei den kapselbildenden Parasiten um Organismen, die zur Gattung *Trichinella* (Trichinen) gehören. In einer anderen bevorzugten Ausführungsform ist die *Trichinella-*Art ausgewählt aus der Gruppe bestehend aus: *Trichinella britovi, Trichinella murrelli. Trichinella nativa, Trichinella nelsoni,* und *Trichinella spiralis.* In einer anderen Ausführungsform sind die nicht-kapselbildenden Parasiten *Trichinella pseudospiralis.* In einer weiteren Ausführungsform des erfindungsgemäßen Verfahrens werden im Wesentlichen intakte Sarcocystis spp. nachgewiesen. Bei den nachzuweisenden Organismen kann es sich um Trichinen, wie z.B. die Arten *Trichinella spiralis, Trichinella britovi*, *Trichinella pseudospiralis, Trichinella nativa, Trichinella murrelli, Trichinella nelsoni, Trichinella papuae* und *Trichinella zimbabwensis handeln.*

Wie in den Beispielen illustriert, stellt die vorliegende Erfindung ein Verfahren bereit zum Nachweis von im Wesentlichen intakten kapselbildenden oder nicht-kapselbildenden Parasiten in Fleisch, das die Mazeration des Fleisches mit einer basischen Verdauungslösung unter Anwesenheit einer im basischen Milieu aktiven Protease umfasst, wobei die Verdauungslösung einen pH-Wert zwischen pH 7,3 und 10 hat, wobei das zu untersuchende Fleisch bevorzugt Fleisch von Schlachttieren oder Fleisch von Tieren, die präpariert werde sollen, ist. Wie hierin verwendet, bezieht sich der Begriff "Fleisch" insbesondere auf Fleisch von untersuchungspflichtigen Schlachttieren, wobei der Begriff "untersuchungspflichtige Schlachttiere" dem Fachmann allgemein bekannt ist. Im weiteren Sinne kann das zu untersuchende Fleisch aber auch vorzugsweise von Säugern, Fischen, Reptilien, Vögeln oder Amphibien stammen. Somit kann das zu untersuchende Fleisch z.B. von Hausschweinen, Rehen, Rindern, Hirschen, Gämsen, Elchen, Wildschweinen, Einhufern, Bären, Füchsen, Biberratten (Nutria), Marderhunden (*Nyctereutes procyonoides*)*,* Straussenvögeln, Krokodilen, Pferden sowie Schlachtvieh bzw. Schlachttieren, Haustieren und Wildtieren wie Haarwild, Federwild, Schalenwild, Schwarzwild, Hochwild, Niederwild, Rehwild, Raubwild, Großwild, Ballenwild oder auch Dachsen stammen. Das Fleisch kann aber auch von allen anderen Tieren stammen, die von kapselbildenden oder nicht-kapselbildenden Parasiten infiziert sein können. Das Fleisch kann auch von anderen Tieren stammen, die Träger von Parasiten des Stammes der *Nematoda* (Fadenwürmer), insbesondere Träger von Trichinen, wie *Trichinella,* sind bzw. von diesen infiziert sind.

Die vorliegende Erfindung umfasst außerdem die Verwendung einer im basischen Milieu aktiven Protease wie einer Endopeptidase, insbesondere einer Serin-Endopeptidase, in einem der voran beschriebenen Verfahren. Insbesondere umfasst die vorliegende Erfindung demnach ein Verfahren zum Nachweis von kapselbildenden oder nicht-kapselbildenden Parasiten in Fleisch, das die (Verdauungs-)Mazeration des Fleisches mit einer basischen Verdauungslösung umfasst, die eine Serin-Endopeptidase enthält.

Die Erfindung betrifft somit auch die Verwendung einer im basischen Milieu aktiven Protease in einem hier beschrieben Verfahren. Das im basischen Milieu aktive Verdauungsenzym kann eine Endopeptidase, vorzugsweise eine Serin-Endopeptidase, sein. Diese Serin-Endopeptidase umfasst ebenfalls Enzyme der Enzymgruppe der Subtilisine. Das Subtilisin kann ausgewählt sein aus der Gruppe bestehend aus: Alcalase® (Subtilisin Carlsberg) und Alcalase 2,5L®.

Wie voran beschrieben, beruht die erfindungsgemäße Verwendung auf basischen Verdauungslösungen, wie in den Beispielen gezeigt, wobei diese Lösung im basischen Milieu aktive Proteasen, wie Serin-Endopeptidasen, beinhalten kann. Der Fachmann kennt jedoch weitere im alkalischen/basischen Milieu aktive Proteasen wie alkalische Proteasen, wobei es sich in einer bevorzugten Ausführungsform bei diesen alkalischen Proteasen um Alkalische Aminopeptidase (EC 3.4.11), Alkalische Cystein Peptidase (EC 3.4.22) oder Alkalische Metallopeptidase (EC 3.4.24) handelt. Auch diese können erfindungsgemäß eingesetzt werden, wobei sich die bereitgestellten Beispiele auf die bevorzugt eingesetzten Serinproteasen beziehen.

Die vorliegende Erfindung umfasst im alkalischen/basischen Milieu aktive Proteasen, wie z. B. eine Serin-Endopeptidase, wie Subtilisin/ Alcalase®, in der Verwendung in einem der hierin beschriebenen Diagnoseverfahren. Daher betrifft die Erfindung ebenfalls ein im basischen Milieu aktives Verdauungsenzym, eine Serin-Endopeptidase, ein Enzym der Enzymgruppe der Subtilisine, Alcalase® (Subtilisin Carlsberg) oder Alcalase 2,5L® zur Verwendung in einem Diagnoseverfahren zum Nachweis einer Infektion mit kapselbildenden oder nicht-kapselbildenden Parasiten.

Die vorliegende Erfindung betrifft darüber hinaus bevorzugt eine Alcalase® (Subtilisin Carlsberg) oder Alcalase 2,5L® zur Verwendung in einem Verfahren zum Nachweis von im Wesentlichen intakten kapselbildenden oder nicht-kapselbildenden Parasiten in Fleisch, das die Mazeration des Fleisches mit einer basischen Verdauungslösung umfasst.

Schließlich betrifft die vorliegende Erfindung die Verwendung eines Kits, umfassend eine basische Verdauungslösung, wobei die Verdauungslösung einen pH-Wert zwischen pH 7,3 und 10 hat, und eine im basischen Milieu aktive Protease, insbesondere eine voran beschriebene Serin-Endopeptidase, zum Nachweis von im Wesentlichen intakten kapselbildenden oder nicht-kapselbildenden Parasiten in Fleisch, das die Mazeration des Fleisches mit einer basischen Verdauungslösung umfasst, die z.B. eine Serin-Endopeptidase enthält. In einer bevorzugten Ausführungsform umfasst das Kit ein Enzym der Enzymgruppe der Subtilisine, in einer weiteren bevorzugten Ausführungsform das Enzym Alcalase® (Subtilisin Carlsberg) oder Alcalase 2,5L®. Ebenfalls werden hier Kits beschrieben, die ein im basischen Milieu aktives Verdauungsenzym, eine Serin-Endopeptidase, ein Enzym der Enzymgruppe der Subtilisine, Alcalase® (Subtilisin Carlsberg) oder Alcalase 2,5L® umfassen, wobei diese "Kits" zum Nachweis von im Wesentlichen intakten kapselbildenden oder nicht-kapselbildenden Parasiten in Fleisch eingesetzt werden.

Die Ausführungsformen, die in Zusammenhang mit dem Verfahren der vorliegenden Erfindung offenbart sind, gelten mutatis mutandis auch für das Kit der vorliegenden Erfindung.

Vorteilhafterweise umfasst das Kit der vorliegenden Erfindung ferner eine Verdauungslösung, die wahlweise (a) Kochsalz (NaCl), (b) Fettemulgatoren, (c) Tenside und/oder (d) Hilfsstoffe enthält. Fettemulgatoren umfassen auch, wie voran beschrieben, Detergentien und sind dem Fachmann bekannt. Vorteilhafterweise umfasst das Kit zudem noch weitere Reaktionspuffer, Aufbewahrungslösungen, Waschlösungen und/oder übrige Reagenzien oder Materialien, die benötigt werden für die Durchführung von Verfahren, wie hierin beschrieben, zum Nachweis von kapselbildenden oder nicht-kapselbildenden Parasiten in Fleisch, das die Mazeration des Fleisches mit einer basischen Verdauungslösung, die z.B. ebenfalls eine Serin-Endopeptidase beinhaltet, umfasst.

Zudem kann das Kit der vorliegenden Erfindung in einer weiteren Ausführungsform ebenfalls Mittel zum Nachweis bzw. zur Bestimmung des basischen pH-Wertes umfassen. Wie voran beschrieben, ist dem Fachmann eine Vielzahl an Methoden bekannt und vertraut, um den pH-Wert einer Lösung mit unterschiedlichen Methoden zu ermitteln. Deshalb umfasst das Kit auch die Ausführungsformen, die im Zusammenhang mit dem Verfahren der vorliegenden Erfindung offenbart sind mutatis mutandis.

In einer weiteren Ausführungsform kann das Kit eine Verdauungslösung, die basisch ist, wobei der pH-Wert dieser Verdauungslösung einen Wert zwischen pH 7,3 und 10 hat, umfassen. In einer Ausführungsform hat die Verdauungslösung einen pH-Wert zwischen pH 7,3 und 9. Wie voran beschrieben wird erfindungsgemäß eine Verdauungslösung mit einem pH-Wert zwischen pH 7,3 und 10 verwendet.

Der pH-Wert der Verdauungslösung ist vorzugsweise größer als pH 7.5, 7.8, 8.0 oder 8.5. Wie in den Beispielen gezeigt kann die Verdauungslösung einen pH zwischen pH 7,3 und 9 haben, z. B. pH 8.0 oder pH 8.5. Andere pH-Werte der Verdauungslösung sind z. B. 7.5, 7.8, 8.0, 8.5, 9.0 oder 9.5. In einer weiteren bevorzugten Ausführungsform wird, je nach eingesetztem Enzym, ein basischer pH-Bereich gewählt, in dem das pH-Optimum des jeweils eingesetzten, im basischen Milieu aktiven Enzyms, liegt.

In einer Ausführungsform umfasst das Kit eine basische Verdauungslösung, wobei der darin beschriebene Emulgator/Fettemulgator z.B. Supralan UF® ist. Der Fettemulgator kann aber auch, wie voran beschreiben, ein Detergens sein oder Detergentien umfassen. Detergentien sind dem Fachmann bekannt und können die oben beschriebenen umfassen. In einer weiteren Ausführungsform kann das Kit der vorliegenden Erfindung eine Verdauungslösung enthalten, wobei die beschriebenen Hilfsstoffe ausgewählt sind aus der Gruppe bestehend aus: Coenzymen, Enzymsubstraten und Katalysatoren. In einer weiteren Ausführungsform kann diese Verdauungslösung Farbstoffe oder färbende Mittel enthalten, mit denen die nachzuweisenden Parasiten angefärbt werden können. In einer solchen Ausführungsform erleichtern die auf diese Weise angefärbten Parasiten den optisch-visuellen Nachweis der Parasiten in den Proben und/oder die Typisierung der Parasiten.

Das Kit der vorliegenden Erfindung kann vorteilhafterweise unter anderem dazu verwendet werden, den Nachweis von im Wesentlichen intakten kapselbildenden oder nichtkapselbildenden Parasiten in Fleisch zu ermöglichen, der die Mazeration des Fleisches mit einer basischen Verdauungslösung umfasst, die z.B. eine Serin-Endopeptidase enthält, wobei die Verdauungslösung einen pH-Wert zwischen pH 7,3 und 10 hat.

Dieses Kit kann also, in Übereinstimmung mit dem Vorstehenden, in einem Verfahren zum Nachweis von kapselbildenden oder nicht-kapselbildenden Parasiten in Fleisch verwendet werden, das die Mazeration des Fleisches mit einer Verdauungslösung umfasst, die z.B. eine Serin-Endopeptidase enthält, optional folgende Schritte umfasst: (a) das mechanische Zerkleinern des zu untersuchenden Fleisches; (b) die Mazeration des zu untersuchenden Fleisches durch Zugabe einer Verdauungslösung; (c) Mazeration unter gleichzeitiger Bewegung des Verdauungsansatzes und/oder gleichzeitiger Ultraschallbehandlung; (d) Inaktivierung der Mazeration; (e) Filtration des Mazerats; und (f) Kontrolle, Nachweis, Diagnostik und oder Typisierung auf Befall mit Parasiten. Wie oben beschrieben kann das mechanische Zerkleinern und die Mazeration gleichzeitig durchgeführt werden. Allerdings kann zunächst das zu untersuchende Fleisch zerkleinert werden und der enzymatische Verdauungsschritt/ die Mazeration findet hinterher statt.

Die Herstellung des Kits folgt bevorzugt Standardprozeduren, die dem Fachmann auf dem Gebiet bekannt sind. Das Kit der vorliegenden Erfindung ist bevorzugt nützlich in einem wie hierin bereitgestellten Verfahren.

Mit dem hierin beschriebenen Verfahren ist es möglich, die Proben innerhalb sehr kurzer Zeit zu verdauen. Eine Verdauung des zu untersuchenden Fleisches ist in ca. 20 min. möglich, ohne an diese Zeit limitiert zu sein. Dies ist, inter alia, in den anhängenden Beispielen gezeigt. Das hier dargestellte Verfahren zum Nachweis von im Wesentlichen intakten kapselbildenden oder nicht-kapselbildenden Parasiten in Fleisch ist somit deutlich schneller als die Referenznachweismethode mit Pepsin, die ca. 40 min benötigt.

Der erfindungsgemäße gesamte Vorgang (Verdauung und Sedimentation) ist etwa gleichlang wie die Verarbeitung im Trichomaten, einer weiteren im Stand der Technik gängigen Methode. Im Trichomaten können allerdings maximal 35 Proben pro Durchgang verarbeitet werden, im Gegensatz zu dem hierin beschriebenen Verfahren, das weit mehr Proben, z.B. 100 Proben pro Durchgang verarbeiten kann.

Mit dem hierin beschriebenen Verfahren geht also sowohl ein Zeitvorteil als auch ein Mengenvorteil einher. Mit dem hierin beschriebenen Verfahren kann mit einer relativ geringen Laborausrüstung die Mazeration durchgeführt werden. Die Proben können nach der Verdaureaktion mit der im Stand der Technik bekannten Sedimentationsmethode mit dem klassischen Scheidetrichter untersucht werden.

Eine Automatisierung zur rationellen Trichinenkontrolle in Grossbetrieben, analog zu dem im Stand der Technik anerkannten automatischen Verfahren mit dem Trichomaten, kann in Übereinstimmung mit der vorliegenden Erfindung durchgeführt werden.

Mit dem hierin beschriebenen Verfahren sind die Materialkosten bedeutend geringer. Da zudem eine größere Probenmenge in einem Durchgang verarbeitet werden kann, ist auch der Personalaufwand geringer.

In den nachfolgend beschriebenen Beispielen wird ein Enzym aus der Enzymgruppe der Subtilisine eingesetzt, wobei die hier beschriebene Erfindung nicht auf dieses Enzym beschränkt ist. Subtilisin ist der Name einer Gruppe von Enzymen, die zur weiteren Gruppe der Serinproteasen/Serinendopeptidasen (Enzymklassifikation EC-Kategorie EC3.4.21) gehört und deren katalysierte Reaktion daher die Spaltung von Proteinketten ist. Zu den Serinproteasen/Serinendopeptidasen gehören unter anderem die Enzyme Chymotrypsin (EC3.4.21.1), Trypsin (EC3.4.21.4), Elastase (EC3.4.21.11), Plasmin (EC3.4.21.7). Thrombin (EC3.4.21.5), Savinase (EC3.4.21.14) und Subtilisin (EC3.4.21.62). Subtilisine kommen hauptsächlich in Bakterien der Gattung *Bacillus* vor und werden seit Jahrzehnten industriell in Waschmitteln verwendet. Eine große Anzahl verschiedener Subtilisine und ähnlicher Enzyme werden aus Bakterien der Gattung *Bacillus* isoliert, insbesondere den Arten *B. subtilis* (Subtilisin E), *B. lentus, B. licheniformis* (Subtilisin Carlsberg, Alcalase®), *B. amyloliquefaciens,* aber auch aus den Schimmelpilzen *Tritirachium album, Thermoactinomyces vulgaris.* Alle diese Mikroorganismen kommen natürlich im Erdboden vor und die Subtilisine dienen ihnen zum Abbau von Proteinen außerhalb der Zelle. Das Enzym ist unter dem Namen Subtilisin Carlsberg bekannt und unter dem Namen Alcalase® registriert und patentiert. Alcalase® ist eine Protease, die benutzt wird, um proteinbasierte Flecken zu entfernen. Sie wird hauptsächlich in Waschmitteln für Textilien und in Geschirrwaschmitteln eingesetzt. Alcalase® wird nur in einer Aktivität hergestellt. Sie wird in flüssiger Form, als Pulver und als Granulat hergestellt. Die Verwendung der flüssigen Form ist in der vorliegenden Erfindung bevorzugt, da Stäube allgemein und Enzym-Stäube im Besonderen als gesundheitsgefährdend gelten. Der verwendete Typ Alcalase 2.5L® wird aus gentechnisch veränderten Bakterien gewonnen. Es gibt andere Alcalasetypen, die aus nicht gentechnisch veränderten Bakterien gewonnen werden. Diese sind bedeutend teurer und werden in Produkten eingesetzt, die nur aus nicht gentechnisch veränderten Organismen hergestellt sein dürfen. Weiter gibt es noch sogenannte "Ultra"-Typen, die zusätzlich vorstabilisiert sind, um besser mit anderen Enzymtypen gemischt werden zu können. In Übereinstimmung mit dem vorstehenden umfasst die vorliegende Erfindung bevorzugt Subtilisine. Die vorliegende Erfindung umfasst nicht nur die vorstehend beschriebenen Subtilisine, sondern auch weitere Subtilisine, die im folgenden beispielhaft Subtilisine genannt sind: Proteinase K; Proteinase R; Proteinase T (isoliert aus Tritirachium album Limber); Subtilisin DY, auch Subtilisin Carlsberg, Subtilisin A, Subtilopeptidase A oder Alcalase Novo genannt; BPN', auch Nagarase Proteinase, Nagarase oder Subtilopeptidase C genannt; Novo, auch Bacterial Proteinase Novo, Subtilisin B, oder Subtilopeptidase B genannt; Mesentericopeptidase und Thermitase. Wie voran beschrieben, beruht die erfindungsgemäße Verwendung auf basischen Verdauungslösungen, wie in den Beispielen gezeigt, wobei diese Verdauungsenzyme wie Serinendopeptidasen beinhalten. Der Fachmann kennt jedoch weitere im alkalischen/basischen Milieu aktive Verdauungsenzyme wie alkalische Proteasen, wobei es sich in einer bevorzugten Ausführungsform bei diesen alkalischen Proteasen um Alkalische Aminopeptidase (EC 3.4.11), Alkalische Cystein Peptidase (EC 3.4.22) oder Alkalische Metallopeptidase (EC 3.4.24) handelt. Auch diese können erfindungsgemäß eingesetzt werden, wobei sich die bereitgestellten Beispiele auf die bevorzugt eingesetzten Serinproteasen beziehen.

Wie vorstehend diskutiert und in den nachstehenden Beispielen illustriert, wird in einer bevorzugten Ausführungsform der Nachweis von im Wesentlichen intakten kapselbildenden oder nicht-kapselbildenden Parasiten in Fleisch so geführt, indem die Mazeration des Fleisches mit einer basischen Verdauungslösung, die eine Serin-Endopeptidase enthält, unter gleichzeitiger Ultraschallbehandlung erfolgt. Wie nachfolgend illustriert, wird durch den Einsatz von Ultraschall die Verdauungsgeschwindigkeit sehr deutlich gesteigert. Durch die Beschallung der Flotte mit Ultraschall ist es möglich, die Proben innerhalb von ca. 15 Minuten zu verdauen. So ist es möglich, in großen Betrieben auch große Probenmengen rasch zu verarbeiten.

Wie in den nachfolgend beschriebenen Beispielen illustriert, kann in einer Ausführungsform der vorliegenden Erfindung normales Leitungswasser verwendet werden. Im UltraschallBad wurde festgestellt, dass eine weiße "Ausfällung" stattfindet, bei der es sich um Kalk handelt. Falls die mikroskopische Auswertung durch die Kalk-Ausfällung gestört werden würde, kann in einer bevorzugten Ausführungsform für die Verdauungsflotte auf destillatgleiches Wasser zurückgegriffen werden.

In einer weiteren bevorzugten Ausführungsform ist bei der Verdauungsreaktion ein pH-Bereich von pH 7.3 bis 9 bevorzugt, einem pH-Bereich, in dem das Optimum der Alcalase® liegt. Die bevorzugte Temperatur mit der besten Aktivität liegt bei ca. 55°C. Da es mit dem Einbringen der Proben in die Verdauungsflotte zu einer Abkühlung kommt, kann in einer weiteren Ausführungsform mit einer initialen Ausgangstemperatur von ca. 60°C gearbeitet werden. Wie voran bereits beschrieben, wird erfindungsgemäß eine Verdauungslösung mit einem pH-Wert zwischen pH 7,3 und 10 verwendet.

Der pH-Wert der Verdauungslösung ist vorzugsweise höher als pH 7.3, 7.5, 7.8, 8.0 oder 8.5. Wie in den Beispielen gezeigt kann die Verdauungslösung einen pH-Wert zwischen pH 7,3 und 9 haben, z. B. pH 8.0 oder pH 8.5. Andere pH-Werte der Verdauungslösung sind z. B. 7.5, 7.8, 8.0, 8.5, 9.0 oder 9.5. In einer weiteren bevorzugten Ausführungsform wird, je nach eingesetztem Enzym, ein basischer pH-Bereich gewählt, in dem das pH-Optimum des jeweils eingesetzten, im basischen Milieu aktiven Enzyms liegt.

Der Begriff "Flotte", wie hierin verwendet, umfasst die Mazerationslösung inklusive aller Zusatzstoffe. Unter dem Begriff "Flottenverhältnis", wie hierin verwendet, wird das Gewichtsverhältnis der Mazerationslösung zu der zu verdauenden Fleischmenge verstanden.

Der Begriff, "Flottenverhältnis", wie hierin verwendet, umfasst auch, dass durch Einsatz von mehr Enzym die Verdauungs-Geschwindigkeit beschleunigt wird. Dabei ist das Verhältnis Fleisch (zu verdauende Probenmenge) zum Enzym entscheidend. Bei einem zu geringen Flottenverhältnis Fleisch zur Verdauungsflüssigkeit kommt es bei der Probenzugabe (Raumtemperatur) zur Verdauungsflotte (60°C) zu einer zu starken Abkühlung der Flotte.

In einer weiteren bevorzugten Ausführungsform wird das hierin beschriebene Verfahren unter Bewegung der Flotte während des Verdauvorganges durchgeführt, da püriertes Fleisch zum einen Teil sedimentiert und zum anderen Teil auf der Flotte aufschwimmt.

Wie voran diskutiert, werden in einer Ausführungsform der vorliegenden Erfindung ebenfalls zusätzlich Emulgatoren, wie Fettemulgatoren, verwendet, da mit den Fleischproben auch immer Fettgewebe in die Verdauungsflotte eingebracht wird. Der Fettanteil ist bei älteren Tieren deutlich höher als bei jungen Tieren. Ohne die Anwendung von Fettemulgatoren schwimmt dieses Fett nach der Verdauung in Form von Fettaugen auf der Wasseroberfläche. Da dieses Fett bei der Zwangsfiltrierung im Filter hängen bleibt, kann es die Untersuchung unter dem Mikroskop beeinträchtigen. Wie in den nachfolgend dargestellten Beispielen illustriert, wird durch den Einsatz von Fettemulgatoren das vorhandene Fett in Lösung gebracht. Somit kann also in einer weiteren Ausführungsform des hier beschrieben Verfahrens und der hier beschriebenen Verwendungen aber auch ein Emulgator/Fettemulgator in der Mazerationslösung/der basischen Verdauungslösung enthalten sein, obgleich die erfindungsgemäßen basischen Verdauungslösungen, wie in den Beispielen gezeigt, keinen Fettemulgator enthalten müssen. Somit können erfindungsgemäß Verdauungslösungen eingesetzt werden, um die potentiell auftretenden negativen Eigenschaften des Fettanteils des zu untersuchenden Fleisches zu verhindern. Der zusätzliche Einsatz von Emulgatoren, wie Fettemulgatoren, in den erfindungsgemäßen Verfahren und Verwendungen und auch den Kits hat den Vorteil, dass das erfindungsgemäße Verfahren nicht behindert wird, sollten diese Fettanteile auftreten. Somit umfasst die vorliegende Erfindung auch im Hinblick auf die hier bereitgestellten Beispiele eine basische Verdauungslösung, die weiterhin einen Emulgator/Fettemulgator beinhaltet. Die Emulgatoren/Fettemulgatoren, die eingesetzt werden, sollten mit dem Enzym kompatibel sein.

Als Emulgator/Fettemulgator kann ein Fettalkoholethoxylat eingesetzt werden. In einer weiteren Ausführungsform wird das Fettalkoholethoxylat mit dem Produktnamen "Supralan UF®" (Produktenummer 2209) der Firma Zschimmer und Schwarz aus Lahnstein/Deutschland eingesetzt. Supralan UF® stammt aus dem Gerbereibereich und hat sehr gute Emulgierfähigkeiten bereits bei geringen Konzentrationen. Es wird als Entfettungsmittel für stark naturfetthaltige Ware eingesetzt und ist chemisch gesehen ein Fettalkoholethoxylat, das flüssig, farblos und nichtionisch ist.

Der Kit kann somit eine im basischen aktive Protease z.B. eine Serinendopeptidase, wie z.B. Subtilisin/Alcalase® enthalten. Der Kit kann außerdem eine basische Verdauungslösung enthalten. Der Kit kann zudem ein Emulgator/Fettemulgator/Detergens enthalten. Somit kann ein Kit der vorliegenden Erfindung eine hierin beschriebene basische Verdauungslösung und ein hierin beschriebenes im basischen Milieu aktiven Verdauungsenzyms enthalten. Ein Kit der vorliegenden Erfindung kann eine hierin beschriebene basische Verdauungslösung und ein hierin beschriebenes Detergens bzw. Emulgator/Fettemulgator enthalten. Außerdem kann ein Kit ein hierin beschriebenes im basischen Milieu aktiven Verdauungsenzym und ein hierin beschriebenes Detergens bzw. Emulgator/Fettemulgator enthalten. Ein weiterer Kit der vorliegenden Erfindung kann eine hierin beschriebene basische Verdauungslösung, ein hierin beschriebenes im basischen Milieu aktiven Verdauungsenzyms, und ein hierin beschriebenes Detergens bzw. Emulgator/Fettemulgator enthalten. Ein Kit der vorliegenden Erfindung kann somit einzelne Bestandteile als auch Kombinationen dieser Bestandteile enthalten, wobei der Kit zur Durchführung des erfindungsgemäßen Verfahrens verwendet wird.

Die basische Verdauungslösung, welche im erfindungsgemäßen Verfahren eingesetzt wird, kann auch Salze, wie z.B. NaCl (Kochsalz enthalten. Bei der Präparation von Knochen mit Enzymen wird meist NaCl (Salz) eingesetzt. Dies verhindert die Bildung von Kalkseifen. Dabei dient NaCl auch als Aktivator für die meisten Enzyme. Es wird eine Einsatzmenge von 10 - 20 g pro Liter empfohlen, je nach Wasserhärte (aus: Knochenpräparation, Handbuch für Praktiker von Niederklopfer/Troxler). Der Begriff "Kalkseifen" wie hierin verwendet, bezieht sich auf in Wasser schwer lösliche Calcium- oder Magnesium-Salze, in der Regel von Fettsäuren. Sie bilden sich bei der Verwendung von Seifen in hartem Wasser. Durch die Bildung dieser Kalkseifen wird die Waschwirkung vermindert, da sich die aktive Seifenmenge verringert und sich die Kalkseifen als Verunreinigung auf den zu reinigenden Oberflächen absetzen. Der Bildung von Kalkseifen kann mit Enthärtern, die die Calcium- und Magnesium-Ionen binden, entgegengewirkt werden. Wegen der genannten Probleme enthalten die heute im Haushalt benutzten Reinigungsmittel in der Praxis keine oder wenig Seifen im klassischen Sinne, sondern andere waschaktive Substanzen (Tenside). Die von den Herstellern als Ersatz für "echte" Seife gewählten Mittel bilden wenig bis keine Kalkseifen und führen so nicht zu Verflockung und schlechter Waschwirkung. Die Bildung von kleineren Mengen an Kalkseifen wird in modernen Waschmitteln genutzt, um eine übermäßige Entwicklung von Schaum zu hemmen. Die Kalkseife entsteht aus den in den Proben vorhandenen tierischen Fetten. In einer speziellen Ausführungsform der vorliegenden Erfindung wird in allen nachfolgend beschriebenen Beispielen 20 g Salz/Liter Flotte eingesetzt, wobei auch andere Salzkonzentrationen verwendet werden können.

Das hierin beschriebene Verfahren kann auch einem Schritt umfassen, in dem die hierin beschriebene Mazeration durch Filtration der Reaktion oder durch Abkühlen der Reaktion terminiert wird. Die Mazeration kann sowohl mechanisch als auch (bio)chemisch terminiert werden. Die Termination besteht z.B. aus Filtration bzw. Abkühlen. Diese Terminierung der Reaktion, wie hierin beschrieben, ist als Inaktivierung der Reaktion zu verstehen. Dies kann von Bedeutung sein, da die Trichinen bei einer zu langen Verweildauer in der Verdauungsflotte auch geschädigt werden können. Deshalb kann nach der geplanten Verdauungszeit der Prozess gestoppt werden. Dies kann in einer bevorzugten Ausführungsform entweder durch eine sofortige Filtration oder durch eine Abkühlung, z. B. Verdünnen mit kaltem Wasser oder einer 0.1%-igen NaCl-Lösung erfolgen.

Der Begriff "Faszie", wie hierin beschrieben, bezeichnet die Weichteil-Komponenten des Bindegewebes, die den ganzen Körper als ein umhüllendes und verbindendes Spannungsnetzwerk durchdringen. Hierzu gehören alle kollagenen faserigen Bindegewebe, insbesondere Gelenk- und Organkapseln, Sehnenplatten (Aponeurosen), Muskelsepten, Bänder, Sehnen, Retinacula (sogenannte "Fesseln" beispielsweise an den Füßen), sowie die "eigentlichen Faszien" in Gestalt von flächigen festen Bindegewebsschichten, wie die Plantarfaszie an der Fußsohle. Vorzugsweise, wie voran diskutiert und in den nachfolgenden Beispielen illustriert, benötigt es eine längere Zeit, bis die Faszien in der Mazerationsflotte aufgelöst sind. Erfolgt die Auflösung der Faszien nicht vollständig, kann die Mazerationsflotte in einer Ausführungsform dieser Erfindung durch einen Grobfilter abgesiebt werden.

### Die Abbildungen zeigen:

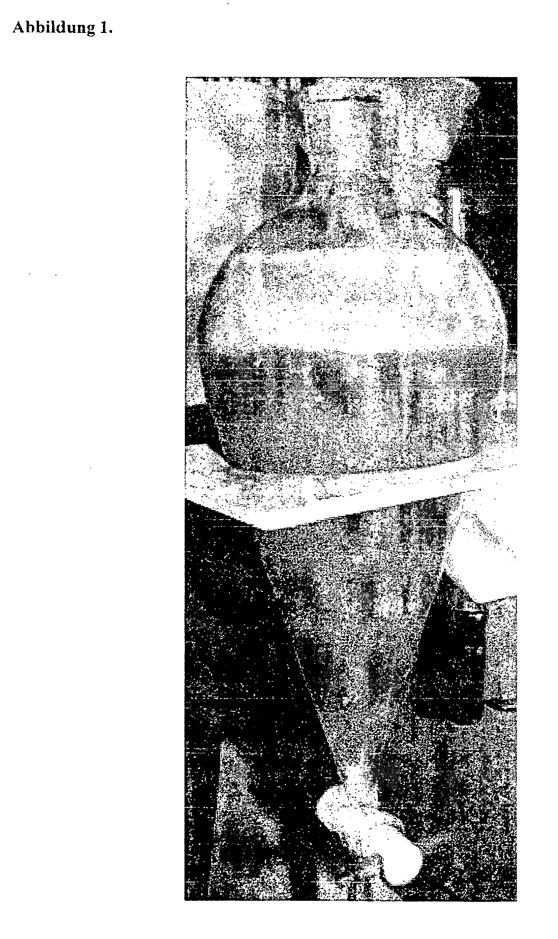

**Abbildung 1****:** Verdauungslösung in einem Scheidetrichter nach 10 min Sedimentationsphase. Eine flockige Ausfällung ist zu beobachten. Die Ausfällung schwimmt nach oben auf und verhindert so eine vollständige Sedimentation der nachzuweisenden Parasiten (siehe Beispiel 3, Versuche 1 und 2). In diesen Versuchen wurde kein Fettemulgator Supralan UF® eingesetzt.

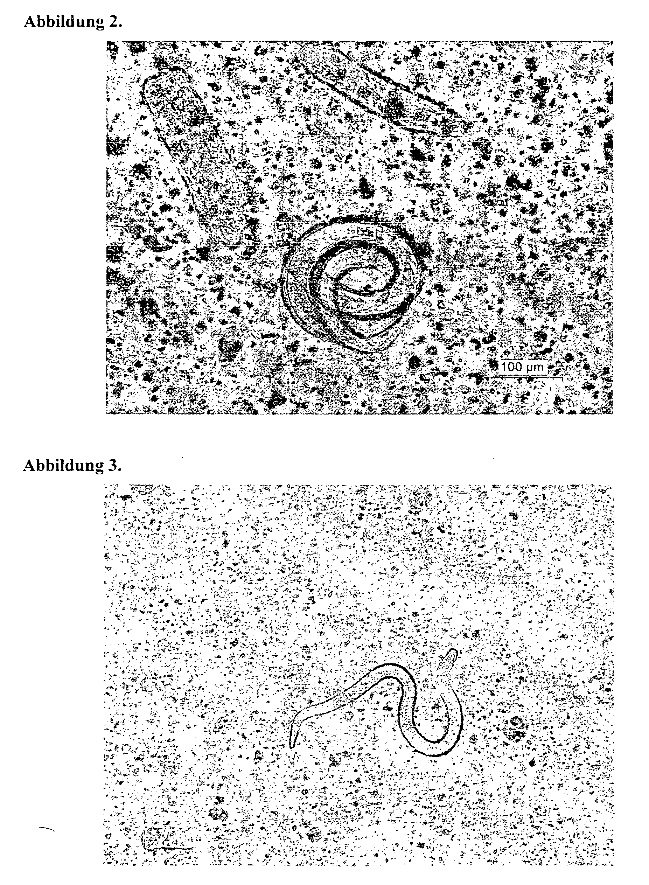

**Abbildung 2****:** Exemplarische Larven in einer Petrischale nach Alcalase®-Verdauung, wie in Beispiel 4.1 beschrieben.
**Abbildung 3****:** Exemplarische Larven in einer Petrischale nach Alcalase®-Verdauung, wie in Beispiel 4.1 beschrieben.

Die vorliegende Erfindung wird durch die folgenden nicht beschränkenden Beispiele veranschaulicht.

### BEISPIELE

### BEISPIEL 1: Material und Methoden

### Material:

Fleischwolf Alexanderwerk 5 zur Grobzerkleinerung des Probenmaterials (Lochgrösse 4 mm)
Becherglas 1200 ml
Becherglas 200 ml
Probenröhrchen
Weithalsflaschen PVC 100 ml
Pürierstab Philips Cucina HR 1350/1351/80/BC mit Messereinsatz
Ultraschallgerät Typ Bandelin Sonorex RK 1028H mit thermostatgesteuertem Wasserbad
Magnetrührer Pyro-Mag Stir Cat. Nr. 34534-200
Digitale Waage Mettler PP200
Digitales Thermometer Oregon Scientific SA880SSX
Stoppuhr Sportcount Combination
pH-Messstreifen Macherey - Nagel 92122
Scheidetrichter konisch, ohne Teilung, mit PTFE-Küken, 1000 ml Lenz-Laborglas, Art. No: 4.0041.7 mit Halterung
Grobfilter Typ Kaffeesieb, Maschengrösse geschätzt 0.8 mm
20 ml Spritze

Leitungswasser
Destillatgleiches Wasser
NaCl
Enzym Subtilisin Carlsberg (Alcalase®)
Enzym Papain 6.000 USP U/mg (= 1,18 mU BAPA/mg)
Emulgator Fettalkoholpolyglycolether (Supralan UF®)
Natriumcarbonat (Soda)

### Methode:

Die zu untersuchende Fleischprobe von 100 g wird im Fleischwolf grob zerkleinert. Ein Becherglas 1200 ml wird mit 500 ml auf 62°C vorgewärmtem destillatgleichem Wasser gefüllt. 5 g NaCl sowie 5 bis 20 g Subtilisin Carlsberg werden beigegeben. Danach wird die Fleischprobe zugegeben. Es folgt eine Durchmischung und weitere Zerkleinerung mit dem Pürierstab während 4 mal 2 Sekunden. Die so vorbereitete Probe wird entweder in das auf 62 °C vorgeheizte und eingeschaltete Ultraschallwasserbad gestellt oder auf dem Magnetrührer weiterverarbeitet. Durch Zugabe von Natriumcarbonat wird der pH-Wert unter Überprüfung mittels pH-Messstreifen auf 8,5 +/- 0.2 eingestellt. Nach der gewünschten Inkubationszeit wird die Flotte durch ein Grobsieb in einen Scheidetrichter umgefüllt. Der Prozess wird durch Beigabe von kalter 0.1 %iger NaCl-Lösung bis zu einem Gesamtvolumen von 1000 ml gestoppt. Die NaCl-Lösung wurde vorher als Spüllösung für das Reaktionsgefäss und den Grobfilter verwendet, um kein Probenmaterial zu verlieren. Nach einer Sedimentationszeit von 10 min wird das Sediment mit 20% der Flüssigkeit in einem Becherglas 200 ml aufgefangen. Der Inhalt des Becherglases wird wiederum in einen Scheidetrichter umgefüllt und mit 400 ml 0.1%iger kalter NaCl-Lösung aufgefüllt. Nach einer zweiten Sedimentation von 10 min wird wiederum das Sediment mit 100 ml der Flüssigkeit in einem Probenröhrchen aufgefangen. Nach einer dritten Sedimentation von weiteren 10 min im Probenröhrchen wird die überstehende Flüssigkeit bis auf 20 ml mit einer Spritze abgesaugt. Das verbleibende Sediment wird mit NaCl-Lösung auf 50 ml aufgefüllt und im Labor des Instituts für Veterinärparasitologie in Bern auf Vorhandensein und Menge von Trichinenlarven untersucht.

In ausgewählten Versuchen wurden zudem zeitgleich mit der Serinprotease 4 g des Fettemulgators Supralan UF® beigegeben.

### Ergebnisse:

**I.** In einem Versuch konnten, mit 20 g Subtilisin Carlsberg/l im Ultraschallbad bei 60°C, pH-Wert 8,5 und 10 g NaCl/l, bei einer Inkubation während 30 min, Trichinen nachgewiesen werden (angedaut). DNA der Trichinen war in der PCR noch identifizierbar, was zur Diagnostik und Typisierung unabdingbare Voraussetzung ist.
**II.** In einem weiteren Versuch wurde eine Probe mit einer bekannten Anzahl Parasiten mit Zwerchfell von Mutterschweinen auf 100 g Probenmenge aufgefüllt, anschliessend durch einen Fleischwolf gedreht und in 500 ml Wasser püriert. Unter Zugabe von 5 g NaCl, 20 g Subtilisin Carlsberg und 4 g Supralan UF® wurde das Ganze auf eine Reaktionstemperatur von 62°C gebracht und mit Soda auf einen pH-Wert von 8.5 eingestellt. Nach 40 min auf dem Magnetrührer waren von Auge nur noch wenige Faszienreste erkennbar.

### BEISPIEL 2: Vergleichende Verdauversuche mit trichinenverseuchten Proben mit den Enzymen Alcalase® und Papain unter Einwirkung von Ultraschall bzw. ohne Bewegung der Flotte

Probenmaterial: trichinenverseuchte Maus, gefrostet.
Alcalase® = Subtilisin Carlsberg (gewonnen aus *Bacillus licheniformis)*
Enzym Papain 6.000 USP U/mg (= 1,18 mU BAPA/mg)

Die verdauten Proben wurden im Becher 3x mind. 10 min sedimentiert und jeweils ca. 4/5 der Flotte sorgfältig mit einer Spritze abgesaugt. Anschliessend wurde das Behältnis jeweils mit einer 0,1%-igen NaCl-Lösung aufgefüllt. Die Auswertung der Proben fand an der Universität Bern, Institut für Parasitologie, statt.

### Versuch 1

| | |
|---|---|
| Herkunft des Probenmaterials: | Brust rechts |
| Versuchsbedingungen: | Alcalase®, im Wärmeschrank (= ohne Bewegung der Flotte); 60°C, pH 8,5, |
| | 10 g Salz/Liter Wasser, |
| | 20 g Alcalase®/Liter Wasser |
| Reaktionszeit: | 5.5 Stunden |

Die Mazeration wurde in einem PVC-Behälter 50 ml durchgeführt.

| | |
|---|---|
| Ergebnis: | Trichinen feststellbar (noch intakt, teilweise noch verkapselt), auch noch Muskelfasern vorhanden; DNA der Trichinen noch feststellbar. |

### Versuch 2

| | |
|---|---|
| Herkunft des Probenmaterials | Hinten links |
| Versuchsbedingungen: | Alcalase®, im Ultraschall; 60°C, pH 8,5, |
| | 10 g Salz/Liter Wasser |
| | 20 g Alcalase®/Liter Wasser |
| Reaktionszeit: | 0.5 Stunden |
| Die Mazeration wurde in einem PVC-Behälter 50 ml durchgeführt. | |
| Ergebnis: | Trichinen feststellbar (angedaut), Flüssigkeit schön klar; DNA der Trichinen noch feststellbar. |

### Versuch 3

| | |
|---|---|
| Herkunft des Probenmaterials: | Hinten rechts |
| Versuchsbedingungen: | Papain, im Ultraschall, Probe vorher gekocht; 40°C, pH 8, |
| | 10 g Salz/Liter Wasser |
| | 5 g Papain/Liter Wasser |
| Reaktionszeit: | 0.5 Stunden |
| Die Mazeration wurde in einem PVC-Behälter 50 ml durchgeführt. | |
| Ergebnis: | keine Trichinen feststellbar. |

### BEISPIEL 3: Vergleichende Verdauversuche mit trichinenverseuchten Proben mit dem Enzym Alcalase®

Probenmaterial: Für alle der nachfolgenden Versuche wurden jeweils ca. 10 g Fleisch vom Pferd oder Schwein mit jeweils 40 lebenden Trichinen versetzt (trichinen-verseuchtes Fleisch). Die verdauten Proben wurden im Scheidetrichter 3x mind. 10 min sedimentiert und jeweils ca. 1/5 der Flotte in einen Becher abgelassen. Die Proben wurden jeweils mit einer 0,1%-igen NaCl-Lösung aufgefüllt. Die Auswertung der Proben fand an der Universität Bern, Institut für Parasitologie statt.

### Versuch 1

| | |
|---|---|
| Probenmaterial: | Trichinen-verseuchtes Pferdefleisch; Probe ca. 10 g, zuzüglich ca. 90 g Zwerchfell altes Schwein (total 100 g Fleisch), Zerkleinerung des Fleisches durch Fleischwolf, anschließend in 500 ml destillatgleichem Wasser 62°C püriert. |
| Verdauansatz: | Analog Versuch 1: (Verhältnis Fleisch, Wasser 1:5), 5 g Salz, 20 g Alcalase®, Soda ca. pH 8.5 (pH-Wert am Anfang ca. 10), 4 g Supralan UF®. |

Reaktionsbedingungen wie in Versuch 1, jedoch erfolgte die Inkubation nicht im Ultraschallbad, sondern auf einem Magnetrührer. Dadurch wurde eine intensive Flottenbewegung erzielt. (Anmerkung: bei der Pepsin-Methode ist es eine zugelassene Variante, die Proben mittels eines Magnetrührers intensiv zu bewegen).

| | |
|---|---|
| Reaktionszeit: | 40 min auf Magnetrührer |
| Ergebnis: | Wenige Faszienreste, vergleichbar mit Beispiel 3, Versuch 8; sehr zuverlässiges Ergebnis: 32 von 40 Trichinen gefunden. Im Gegensatz zu einem herkömmlichen Verdau mit Pepsin haben die Trichinen die Verdauung nicht überlebt; dies hat den Vorteil, dass die Proben nicht mehr infektiös sind. |

### Versuch 2

| | |
|---|---|
| Probenmaterial: | Trichinen-verseuchtes Schweinefleisch; Probe ca. 10 g, zuzüglich ca. 90 g Zwerchfell, altes Schwein (total 100 g Fleisch). Zerkleinerung des Fleisches durch Fleischwolf, anschließend in 500 ml destillatgleichem Wasser 62°C püriert. |
| Verdauansatz: | (Verhältnis Fleisch, Wasser 1:5), 5 g Salz, 5 g Alcalase®, Soda ca. pH 8.0. |
| Reaktionszeit: | 30 min unter gleichzeitiger Ultraschallbehandlung. |
| Ergebnis: | 2 Trichinen feststellbar, diese waren intakt und nicht angedaut. |

Eine Sedimentation nach unten und ein Aufschwimmen von Material nach oben ist zu beobachten. Dies erklärt, warum die Sedimentation nicht erfolgreich ist. Die Ausfällung schwimmt nach oben auf und verhindert so eine vollständige Sedimentation der nachzuweisenden Parasiten. Eine Zwangsfiltration (Filtration der Lösung, gegebenenfalls unter Zusatz von Druck oder Vakuum) kann dem entgegenwirken und kann beim Auftreten einer Ausfällung eingesetzt werden.

### BEISPIEL 4:Validierung der erfindungsgemäßen Methode durch das Institut für Parasitologie der Universität Bern

### Material

Waage Mettler Toledo, Modell PG2002-S
Kompressorium (Quetschgläser), bestehend aus zwei gegeneinander drückbaren Glasplatten
Püriergerät Waring Commercial Blender
Becherglas 125 ml
pH-Indikatorstreifen Merck: Alkalit pH 7.5-14
Magnetrührer inkl. Temperatursonde IKA RCT basic
Grobfilter Typ Kaffeesieb, Maschengröße geschätzt 0.8 mm
Scheidetrichter
Zentrifugenröhrchen 10 ml
Petrischale
Stereolupe Leica MS5, 40-fache Vergrößerung
Vakuumfiltrationsgerät aus Duranglas, Filterdurchmesser 45-50 mm
Filtermembrane 14,0 Micron GE Water & Process Technologies, Cat No K14CP05000
Wasserstrahlpumpe

Leitungswasser
NaCl
Enzym Substilisin Carlsberg (Alcalase®)
Emulgator Fettalkoholpolyglycolether (Supralan UF®)
Natriumcarbonat (Soda)

### BEISPIEL 4.1: Vergleichende Verdauversuche mit trichinenverseuchten Proben mit dem Enzym Alcalasc® im Vergleich zu Pepsin

Versuchsdurchführung: Ein Stück Mäusefleisch, infiziert mit Trichinen wird im Kompressorium gepresst und die Larvenzahl bestimmt. Dieser Probe werden 25 g Zwerchfell von Mutterschweinen zugegeben und im Mixer püriert. 125ml H₂O werden im Becherglas auf 55°C erwärmt (Verhältnis Fleisch zu Wasser 1:5). Das pürierte Fleisch wird ins erwärmte Wasser überführt und 2 g NaCl, 0.5 g Natrium-Bicarbonat und 5 ml Alcalase® zugegeben. Mit einem pH-Indikatorstreifen wird der pH-Wert überprüft (Ziel pH 8 bis 8.5). Inkubation bei 55°C auf dem Magnetrührer unter kräftigem Rühren (tiefer zentraler Wirbel). Die Temperatur wird während der ganzen Zeit mittels Sonde überprüft. Nach der Inkubationszeit von 20 min wird die Flotte durch das Sieb in den Scheidetrichter umgefüllt. Der Becher wird mit 125 ml H₂O ausgewaschen und der Inhalt ebenfalls durch das Sieb in den Scheidetrichter gegeben, 30 min sedimentiert und 8 ml des Sedimentes in ein Zentrifugenröhrchen abgelassen, dann nochmals 15 min sedimentiert. Die oberen 6 ml des Inhalts des Zentrifugenröhrchens werden mit einer Pipette abgesaugt und die verbleibenden 2 ml in einer Petrischale unter der Stereolupe bei 40-facher Vergrößerung mikroskopiert.

Parallel dazu wird jeweils die konventionelle Verdauung mit Pepsin mit den gleichen Gerätschaften durchgeführt. Der Rückstand auf dem Sieb wird gewogen. Die Versuche werden immer nach dem gleichen Schema von 3 verschieden Personen durchgeführt. Die Petrischalen werden jeweils von zwei verschiedenen Personen abgelesen. *Trichinella*-Quelle: Maus infiziert mit *T. spiralis.*

### Ergebnisse:

Dauer der Untersuchung: Alcalase®-Verdauung ca. 70 min, Pepsin Verdauung ca. 90 min. Die Alcalase ist ca. 20 min schneller, da die Verdauung 20 min braucht, bei Pepsin 40 min. Rückstand auf Sieb bei beiden Methoden ca. 2%
Alcalase-Verdauung: Ausflockung von Material in der Verdauungslösung, dieses sinkt z. T. ab und schwimmt z. T. in Wolken im Scheidetrichter ohne zu sedimentieren. Durchsichtigkeit des Sedimentes schlechter bei der Alcalase® als beim Pepsin. Bei der Alcalase® sind mehr kleine und kleinste Fasern sichtbar, beim Pepsin eher größere Stückchen, dafür sehr wenige.

Anzahl aufgefundene Larven: bei der Pepsinverdauung 90-95% bei der Alcalaseverdauung 25-50%
Larvenmorphologie: nach der Pepsinverdauung lebende Larven, nach der Alcalase-Verdauung stark eingerollte Larven ohne sichtbare Beweglichkeit. Die Stichozyten sind bei der Pepsin-Verdauung besser sichtbar.

### Beispiel 4.1.1: Parallelverdauung

25 g Schweinefleisch versetzt mit 1 Stück Mausmuskulatur mit 50 Larven.
Pepsin: 49 Larven gefunden. Alcalase®: 19 Larven gefunden.

### BEISPIEL 4.2: Verdauversuche mit trichinenverseuchten Proben mit anschliessender Zwangsfiltration

Es wird vermutet, dass die schlechte Sensitivität auf eine ungenügende Sedimentation zurückzuführen ist. Aus diesem Grund werden die Versuche analog der Versuche, wie unter Beispiel 4.1 beschrieben, durchgeführt, allerdings wird die Verdauungsflüssigkeit in einem Filterapparat mit einer Filtermembrane von 14,0 Micron unter Zuhilfenahme einer Wasserstrahl-Vakuum-Pumpe zwangsfiltriert.

### Ergebnisse:

Auf der Filtermembran sind einige Larven erkennbar.

### BEISPIEL 4.3: Verdauversuche mit trichinenverseuchten Proben: Kombination aus Sedimentation und Zwangsfiltration

Aufgrund der schlechten Lesbarkeit der in Beispiel 4.2 beschriebenen Versuche wird eine Kombination aus Sedimentation und Zwangsfiltration beschlossen.
Überlegung: Wenn zuerst ein Sedimentationsschritt erfolgt, sollte weniger Material im Überstand sein und die Lesbarkeit der Filtermembran sollte dadurch verbessert werden. Weiter wird das Produkt Supralan UF® wieder in die Versuche aufgenommen. Es erfolgt kein Vergleich mit der Pepsin-Verdauung.

### Trichinella-Quelle: Maus infiziert mit T. britovi.

### Versuch 1

Ein mit Trichinen infiziertes Stück Mäusefleisch wird im Kompressorium gepresst und eine Zahl von ca. 42 Larven bestimmt. Dieser Probe werden 13 g Pferdefleisch (Zunge) zugegeben und im Mixer püriert. 125 ml H₂O werden im Becherglas auf 55°C erwärmte (Verhältnis Fleisch zu Wasser ca. 1:10). Das pürierte Fleisch wird ins erwärmte Wasser überführt und 2 g NaCl, 0.5 g Natrium-Bicarbonat, 5ml Alcalase® und 1 ml Supralan UF® zugegeben. Mit einem pH-Indikatorstreifen wird der pH-Wert überprüft (Ziel pH 8 bis 8.5). Inkubation bei 55 °C auf dem Magnetrührer unter kräftigem Rühren (tiefer zentraler Wirbel), während 20 min. Temperatur während der ganzen Zeit mittels Sonde überprüft. Nach der Inkubationszeit wird die Flotte durch das Sieb in den Scheidetrichter umgefüllt. Der Becher wird mit 125 ml H₂O ausgewaschen und der Inhalt ebenfalls durch das Sieb in den Scheidetrichter gegeben, 30 min sedimentiert, 8 ml des Sedimentes in Zentrifugenröhrchen abgelassen und nochmals 15 min sedimentiert. Die oberen 6 ml des Inhalts des Zentrifugenröhrchens werden mit einer Pipette abgesaugt und die verbleibenden 2 ml in einer Petrischale unter der Stereolupe bei 40-facher Vergrößerung mikroskopiert. Die Überstände aus dem Scheidetrichter und dem Zentrifugenröhrchen werden unter Zuhilfenahme einer Wasserstrahl-Vakuumpumpe durch den Filter gezogen.

### Ergebnis:

In Petrischale 42 Larven, auf Filter 3 Larven; Lesbarkeit der Petrischale gut, Filter immer noch schwierig zu lesen, aber deutlich besser als bei den Versuchen mit Filtration der gesamten Verdauungslösung.

### Versuch 2

Analog Versuch 1, außer: 10 Larven im Kompressorium bestimmt und 17 g Zwerchfell von Mutterschweinen statt Pferdefleisch.

### Ergebnis

In Petrischale 10 Larven. Filter durch zu starkes Vakuum zerrissen.

### Versuch 3

Analog Versuch 2, außer 20 Larven im Kompressorium bestimmt und 18 g Zwerchfell von Mutterschweinen.

### Ergebnis

In Petrischale 20 Larven. Kein Filtrationsschritt durchgeführt.

Bei allen 3 Versuchen ist die Verdauung seit dem Zusatz von Supralan UF® bedeutend besser, und dadurch sind unter dem Mikroskop deutlich weniger Faserreste zu sehen, die die Untersuchung stören. Die Sedimentation funktioniert seit dem Einsatz von Supralan UF®.

### Analyse und Kommentar zu den Beispielen 3, 4.1 und 4.2

Anhand der Analyse der Versuche ist zu erkennen, dass der Emulgator/Fettemulgator Supralan UF® eingesetzt werden kann, falls die beschriebenen Sedimentationen oder Ausfällungen auftreten. Der Einsatz eines Emulgators/Fettemulgators kann also das Ergebnis gegenüber einem Ansatz ohne Emulgator/Fettemulgator verbessern bzw. den Nachweis zusätzlich erleichtern.

### BEISPIEL 4.4: Verdauversuche mit trichinenverseuchten Proben: Vergleich Alcalase®-Verdauung mit Pepsin-Verdauung anhand trichinenverseuchter Proben unterschiedlicher Herkunft

Ziel: Nachweis, dass mit der Alcalase®-Verdauung mindestens die gleiche Sensitivität erreicht werden kann, wie mit der Pepsin-Verdauung. Dies mit Schweine-, Wildschweine- und Pferdefleisch und für die drei *Trichinella*-Arten *T. spiralis, T. britovi* und *T. pseudospiralis.*
Prinzip: Parallelansätze Alcalase® und Pepsin, unterschiedliche Larvenbelastungen.
Die Versuche werden analog der Versuche 1 bis 3, wie in Beispiel 5.3 beschrieben, durchgeführt, jedoch mit verschiedenen Larvenbelastungen.
Verhältnis Fleisch zu Wasser ca. 1:10.

### Material:

Zunge vom Pferd, Zwerchfell vom Schwein und Wildschwein Larven von *T. spiralis. T. britovi* und *T. pseudospiralis*

### Ergebnisse:

Die Ergebnisse sind in nachfolgender Tabelle zusammengefasst.

| **Probe** | **Alcalase** | | | | **Pepsin** | | | |
|---|---|---|---|---|---|---|---|---|
| | **Larven zugegeben** | **Larven nachgewiesen** | **%** | **Siebrückstand (g)** | **Larven zugegeben** | **Larven nachgewiesen** | **%** | **Siebrückstand (g)** |
| **T. spiralis/Schwein** | 10 | 7 | 70 | 0,01 | 11 | 10 | 91 | 0,45 |
| T. **spiralis/Schwein** | 11 | 8 | 72 | 0,15 | 10 | 10 | 100 | 0,33 |
| T. **spiralis/Schwein** | 24 | 20 | 83,3 | 0,4 | 20 | 16 | 80 | 0,63 |
| T. **spiralis/Schwein** | 20 | 19 | 95 | 0,56 | 21 | 21 | 100 | 0,05 |
| T. **spiralis/Schwein** | 4 | 3 | 75 | 0,2 | 3 | 3 | 100 | 0,57 |
| T. **spiralis/Schwein** | 3 | 3 | 100 | 0,67 | 4 | 4 | 100 | 0,01 |
| | | | | | | | | |
| **T.spiralis/Pferd** | 9 | 7 | 77,8 | 2,4 | 9 | 4 | 44,4 | 0,3 |
| **T.spiralis/Pferd** | 4 | 2 | 50 | 0,67 | | | | |
| | | | | | | | | |
| T. **spiratis/W'schwein** | 7 | 7 | 100 | 1 | 11 | 9 | 81,8 | 0,26 |
| T. **spiralis/W'schwein** | 3 | 3 | 100 | 0,46 | 4 | 3 | 75 | 0,64 |
| | | | | | | | | |
| T. **pseudospiralis/Schwein** | 3 | 1 | 33,3 | 0,24 | 3 | 1 | 33,3 | 2,4 |
| T. **pseudospiralis/Schwein** | 8 | 8 | 100 | 0,9 | 6 | 1 | 16,7 | 0,9 |
| | | | | | | | | |
| T. **britovi/Schwein** | 10 | 10 | 100 | | | | | |
| T. **britovi/Schwein** | 20 | 20 | 100 | | | | | |
| | | | | | | | | |
| **T.britovi/Pferd*** | 40 | 42 | 100 | | | | | |

| | | | | | | | | |
|---|---|---|---|---|---|---|---|---|
| PCR mit Larven von T. spiralis und T. pseudospiralis nach Alcalase®-Verdauung aus den obenstehenden Versuchen haben einwandfrei funktioniert. (*) Aufgrund der Schwierigkeit der Bestimmung der Larvenzahl im Kompressorium kann es sein, dass die Zahl der gefunden Larven wie im Versuch *T.britovi*/Pferd, aufgrund eines Zählfehlers höher ist, als die Zahl der zugegebenen Larven. | | | | | | | | |

## Patentansprüche

1. Verfahren zum Nachweis von im Wesentlichen intakten kapselbildenden oder nicht-kapselbildenden Parasiten in Fleisch, das die Mazeration des Fleisches mit einer basischen Verdauungslösung, die im basischen Milieu aktive Proteasen enthält, umfasst, wobei die Verdauungslösung einen pH-Wert zwischen pH 7,3 und 10 hat.

2. Verfahren nach Anspruch 1, wobei das Verdauungsenzym eine Serin-Endopeptidase ist.

3. Verfahren nach Anspruch 1 oder 2, das die Schritte umfasst:
(a) das mechanische Zerkleinern des zu untersuchenden Fleisches;
(b) die Mazeration des zu untersuchenden Fleisches durch Zugabe einer Verdauungslösung;
(c) Inaktivierung der Mazeration;
(d) Filtration des Mazerats; und
(e) Kontrolle, Nachweis, Diagnostik und/oder Typisierung auf Befall mit Parasiten.

4. Verfahren nach einem der Ansprüche 1 bis 3, wobei das Verhältnis Fleisch/Verdauungslösung vorzugsweise 1:5, 1:10 oder 1:20 ist.

5. Verfahren nach einem der Ansprüche 2 bis 4, wobei die Serin-Endopeptidase vorzugsweise ein Enzym der Enzymgruppe der Subtilisine ist.

6. Verfahren nach Anspruch 5, wobei das Subtilisin rekombinant oder nicht-rekombinant hergestellt ist und/oder ausgewählt ist aus der Gruppe bestehend aus: Alcalase® (Subtilisin Carlsberg) und Alcalase 2,5L®.

7. Verfahren nach einem der Ansprüche 1 bis 6, wobei die Verdauungslösung NaCl enthält und/oder optional Fettemulgatoren, Tenside und/oder Hilfsstoffe enthält.

8. Verfahren nach Anspruch 7, wobei der Fettemulgator Supralan UF® ist.

9. Verfahren nach Anspruch 7, wobei die Hilfsstoffe ausgewählt sind aus der Gruppe bestehend aus Coenzymen, Enzymsubstraten und Katalysatoren.

10. Verfahren nach einem der Ansprüche 1 bis 9, wobei die Mazeration unter gleichzeitiger Ultraschallbehandlung, unter gleichzeitiger Bewegung und/oder gleichzeitigem Rühren mit einem Magnetrührer und/oder bei einer Temperatur zwischen 55°C und 65°C durchgeführt wird.

11. Verfahren nach einem der Ansprüche 1 bis 10, wobei die Inaktivierung der Mazeration durch Filtration der Reaktion oder durch Abkühlen der Reaktion terminiert wird.

12. Verfahren nach einem der Ansprüche 1 bis 11, wobei bei der Filtration des Mazerats die Maschengröße der verwendeten Filter auf die Größe der nachzuweisenden Organismen angepasst wird.

13. Verfahren nach einem der Ansprüche 1 bis 12, wobei die Kontrolle, der Nachweis, die Diagnostik und/oder die Typisierung auf Befall mit Parasiten visuell erfolgt.

14. Verfahren nach einem der Ansprüche 1 bis 13, wobei die kapselbildenden oder nicht-kapselbildenden Parasiten zum Stamm der *Nematoda* (Fadenwürmer) und/oder zur Gattung *Trichinella* (Trichinen) gehören.

15. Verfahren nach einem der Ansprüche 1 bis 14, wobei die *Trichinella*-Art ausgewählt ist aus der Gruppe bestehend aus *Trichinella spiralis, Trichinella britovi, Trichinella pseudospiralis, Trichinella nativa, Trichinella murrelli, Trichinella nelsoni, Trichinella papuae* und *Trichinella zimbabwensis.*

16. Verfahren nach einem der Ansprüche 1 bis 15, wobei das zu untersuchende Fleisch ausgewählt ist aus der Gruppe bestehend aus Schlachttieren, Haustieren, Wildtieren und zur Präparation bestimmte Tiere.

17. Verfahren nach Anspruch 16, wobei das zu untersuchende Fleisch von Schlachttieren, Haustieren, Wildtieren oder der zur Präparation bestimmten Tiere ausgewählt ist aus Fleisch vom Hausschwein, Wildschwein, Bär, Reh, Hirsch, Gämse, Elch, Pferd, Fuchs, Biberratte (Nutria), Marderhund, Dachs, Vogel Strauss und Krokodil.

18. Verwendung einer im basischen Milieu aktiven Protease in einem der Verfahren nach einem der Ansprüche 1 bis 17.

19. Verwendung nach Anspruch 18, wobei das im basischen Milieu aktive Protease eine Endopeptidase, vorzugsweise eine Serin-Endopeptidase ist.

20. Verwendung nach Anspruch 19, wobei die Serin-Endopeptidase ein Enzym der Enzymgruppe der Subtilisine ist.

21. Verwendung nach Anspruch 20, wobei das Subtilisin ausgewählt ist aus der Gruppe bestehend aus: Alcalase® (Subtilisin Carlsberg) und Alcalase 2,5L®.

22. Verwendung eines Kits zum Nachweis von im Wesentlichen intakten kapselbildenden oder nicht-kapselbildenden Parasiten im Fleisch, umfassend eine basische Verdauungslösung und eine im basischen Milieu aktive Protease, wobei die Verdauungslösung einen pH-Wert zwischen pH 7,3 und 10 hat.

## Claims

1. Method for the detection of essentially intact encapsulated or non-encapsulated parasites in meat, comprising maceration of meat with an alkaline digestion solution which contains a protease that is active in an alkaline environment, wherein the digestion solution has a pH-value between pH 7.3 and 10.

2. The method of claim 1, wherein the digestive enzyme is a serine endopeptidase.

3. The method of claim 1 or 2, comprising the steps of:
(a) mechanically grinding the meat to be analysed;
(b) macerating the meat to be analysed by adding a digestion solution;
(c) inactivation of the maceration;
(d) filtration of the macerate; and
(e) control, detection, diagnosis and/or classification regarding parasite infestation.

4. The method of any one of claims 1 to 3, wherein the ratio of meat/digestion solution is preferably 1:5, 1:10 or 1:20.

5. The method of any one of claims 2 to 4, wherein the serine endopeptidase is preferably an enzyme of the enzyme group of the subtilisins.

6. The method of claim 5, wherein the subtilisin is produced recombinantly or non-recombinantly and/or is selected from the group consisting of: Alcalase^{®} (Subtilisin Carlsberg) and Alcalase 2.5L^{®}.

7. The method of any one of claims 1 to 6, wherein the digestion solution contains NaCl and/or optionally contains fat emulsifiers, surfactants and/or auxiliary agents.

8. The method of claim 7, wherein the fat emulsifier is Supralan UF^{®}.

9. The method of claim 7, wherein the auxiliary agents are selected from the group consisting of coenzymes, enzyme substrates and catalysts.

10. The method of any one of claims 1 to 9, wherein the maceration takes place under simultaneous sonication, under simultaneous agitation and/or simultaneous stirring with a magnetic stirrer and/or is carried out at a temperature between 55°C and 65°C.

11. The method of any one of claims 1 to 10, wherein the inactivation of the maceration is terminated by filtration of the reaction or by cooling the reaction.

12. The method of any one of claims 1 to 11, wherein, for the filtration of the macerate, the mesh size of the filters used is adapted to the size of the organisms to be detected.

13. The method of any one of claims 1 to 12, wherein control, detection, diagnosis and/or categorisation of parasite infestation are visual.

14. The method of any one of claims 1 to 13, wherein the encapsulated or non-encapsulated parasites belong to the phylum of *Nematoda* (roundworms) and/or to the genus *Trichinella* (trichina).

15. The method of any one of claims 1 to 14, wherein the *Trichinella* species is selected from the group consisting of *Trichinella spiralis, Trichinella britovi, Trichinella pseudospiralis, Trichinella nativa, Trichinella murrelli, Trichinella nelsoni, Trichinella papuae* and *Trichinella zimbabwensis.*

16. The method of any one of claims 1 to 15, wherein the meat to be analysed is selected from the group consisting of animals for slaughter, domestic animals, wild animals and animals intended for taxidermy.

17. The method of claim 16, wherein the meat to be analysed of animals for slaughter, domestic animals, wild animals or of animals intended for taxidermy is selected from the meat of domestic pig, wild boar, bear, roe deer, red deer, alpine chamois, elk, horse, red fox, nutria, raccoon dog, badger, ostrich and crocodile.

18. Use of a protease that is active in an alkaline environment in one of the methods of any one of claims 1 to 17.

19. The use of claim 18, wherein the digestive enzyme that is active in an alkaline environment is an endopeptidase, preferably a serine endopeptidase.

20. The use of claim 19, wherein the serine endopeptidase is an enzyme of the enzyme group of the subtilisins.

21. The use of claim 20, wherein the subtilisin is selected from the group consisting of: Alcalase^{®} (Subtilisin Carlsberg) and Alcalase 2.5L^{®}.

22. Use of a kit for the detection of essentially intact encapsulated or non-encapsulated parasites in meat, comprising an alkaline digestion solution and a protease that is active in an alkaline environment, wherein the digestion solution has a pH-value between pH 7.3 and 10.

## Revendications

1. Procédé de détection de parasites sensiblement intacts encapsulés ou non encapsulés dans de la viande, comprenant la macération de la viande avec une solution de digestion alcaline qui contient une protéase qui est active dans un environnement alcalin, dans lequel la solution de digestion présente une valeur de pH comprise entre pH 7,3 et pH 10.

2. Procédé selon la revendication 1, dans lequel l'enzyme digestive est une endopeptidase de sérine.

3. Procédé selon la revendication 1 ou 2, comprenant les étapes consistant à :
(a) hacher mécaniquement la viande à analyser ;
(b) faire macérer la viande à analyser en ajoutant une solution de digestion ;
(c) inactiver la macération ;
(d) filtrer la macération ; et
(e) contrôler, détecter, diagnostiquer et/ou classer une infestation de parasite.

4. Procédé selon l'une quelconque des revendications 1 à 3, dans lequel le rapport viande/solution de digestion est de préférence égal à 1/5, à 1/10 ou à 1/20.

5. Procédé selon l'une quelconque des revendications 2 à 4, dans lequel l'endopeptidase de sérine est de préférence une enzyme du groupe d'enzymes des subtilisines.

6. Procédé selon la revendication 5, dans lequel la subtilisine est produite de manière recombinée ou non recombinée et/ou est sélectionnée dans le groupe comprenant : l'Alcalase^{®} (Subtilisine Carlsberg) et l'Alcalase 2.5L^{®}.

7. Procédé selon l'une quelconque des revendications 1 à 6, dans lequel la solution de digestion contient du NaCl et/ou contient éventuellement des émulsifiants gras, des agents tensio-actifs et/ou des agents auxiliaires.

8. Procédé selon la revendication 7, dans lequel l'émulsifiant gras est du Supralan UF^{®}.

9. Procédé selon la revendication 7, dans lequel les agents auxiliaires sont sélectionnés dans le groupe comprenant des coenzymes, des substrats d'enzymes et des catalyseurs.

10. Procédé selon l'une quelconque des revendications 1 à 9, dans lequel la macération se déroule sous une sonification simultanée, sous une agitation simultanée et/ou sous une agitation simultanée avec un agitateur magnétique et/ou est exécutée à une température comprise entre 55 °C et 65 °C.

11. Procédé selon l'une quelconque des revendications 1 à 10, dans lequel l'inactivation de la macération est terminée par une filtration de la réaction ou par un refroidissement de la réaction.

12. Procédé selon l'une quelconque des revendications 1 à 11, dans lequel, pour la filtration de la macération, la taille des mailles des filtres utilisés est adaptée à la taille des organismes à détecter.

13. Procédé selon l'une quelconque des revendications 1 à 12, dans lequel le contrôle, la détection, le diagnostic et/ou la catégorisation de l'infestation de parasite sont visuels.

14. Procédé selon l'une quelconque des revendications 1 à 13, où les parasites encapsulés ou non encapsulés appartiennent à l'embranchement *Nematoda* (vers ronds) et/ou au genre *Trichinella* (trichines).

15. Procédé selon l'une quelconque des revendications 1 à 14, dans lequel les espèces de *Trichinella* sont sélectionnées dans le groupe comprenant *Trichinella spiralis, Trichinella britovi, Trichinella pseudospiralis, Trichinella nativa, Trichinella murrelli, Trichinella nelsoni, Trichinella papuae* et *Trichinella zimbabwensis.*

16. Procédé selon l'une quelconque des revendications 1 à 15, dans lequel la viande à analyser est sélectionnée dans le groupe comprenant des animaux destinés à l'abattage, des animaux domestiques, des animaux sauvages et des animaux destinés à l'empaillage.

17. Procédé selon la revendication 16, dans lequel la viande à analyser des animaux destinés à l'abattage, des animaux domestiques, des animaux sauvages ou des animaux destinés à l'empaillage est sélectionnée parmi la viande de porc domestique, de sanglier, d'ours, de chevreuil, de cerf rouge, de chamois alpin, d'élan, de cheval, de renard roux, de ragondin, de chien viverrin, de blaireau, d'autruche et de crocodile.

18. Utilisation d'une protéase qui est active dans un environnement alcalin dans l'un des procédés selon l'une quelconque des revendications 1 à 17.

19. Utilisation selon la revendication 18, dans laquelle l'enzyme de digestion qui est active dans un environnement alcalin est une endopeptidase, de préférence une endopeptidase de sérine.

20. Utilisation selon la revendication 19, dans laquelle l'endopeptidase de sérine est une enzyme du groupe d'enzymes des subtilisines.

21. Utilisation selon la revendication 20, dans laquelle la subtilisine est sélectionnée dans le groupe comprenant : l'Alcalase^{®} (Subtilisine Carlsberg) et l'Alcalase 2,5L^{®}.

22. Utilisation d'un kit de détection de parasites sensiblement intacts encapsulés ou non encapsulés dans de la viande, comprenant une solution de digestion alcaline et une protéase qui est active dans un environnement alcalin, dans lequel la solution de digestion présente une valeur de pH comprise entre pH 7,3 et pH 10.
